# EUROPEAN PATENT SPECIFICATION

(11) **EP 1 100 488 B1**
(45) Date of publication and mention of the grant of the patent: **23.04.2003**
(21) Application number: 98942645.7
(22) Date of filing: 28.07.1998
(51) Int. Cl.: A61K 31/195, A61P 19/00, A61P 19/10

(54) **USE OF CREATINE COMPOUNDS FOR TREATMENT OF BONE OR CARTILAGE CELLS AND TISSUES**
VERWENDUNG VON KREATINSUBSTANZEN ZUR BEHANDLUNG VON KNOCHEN- UND KNORPELZELLEN UND GEWEBEN
UTILISATION DE COMPOSES A BASE DE CREATINE POUR TRAITER LES CELLULES ET LES TISSUS OSSEUX ET CARTILAGINEUX

(43) Date of publication of application: 23.05.2001
(73) Proprietor: SYNTHES AG Chur, 7002 Chur (CH)
(72) Inventor: WALLIMANN, Theo, CH-8963 Kindhausen (CH); GERBER, Isabel, CH-7260 Davos Dorf (CH)
(74) Representative: Lusuardi, Werther Giovanni, Dr.
(86) International application number: EP9804713
(87) International publication number: WO00006150

(56) References cited:
- EP-A- 0 891 719
- WO-A-97/45533
- SOMJENSOMJEN D ET AL: "Nonhypercalcemic Analogs of Vitamin D Stimulate Creatine Kinase B Activity in Osteoblast-Like ROS 17/2.8 Cells and Up-regulate Their Responsiveness to Estrogens - new actions, new analogs, new therapeutic potential" STEROIDS: STRUCTURE, FUNCTION, AND REGULATION, vol. 63, no. 5-6, 6 May 1998, page 340-343 XP004132223

## Description

This invention concerns the use of creatine compounds in accordance with the pre-characterising portions of claims 1 and 2.

The creatine compounds may be incorporated in three dimensional constructs of osteoblasts, chondrocytes or mesenchymal stem cells designed for tissue engineering of said bone or cartilage defects.

Further the creatine compounds may be used for improving acceptance and osseous integration of bone implants.

Creatine is a compound which is naturally occurring in the human body and is found in mammalian brain and other excitable tissues, such as skeletal muscle, heart and retina. It's phosphorylated form, creatine phosphate is also found in the same organs and is the product of the creatine kinase reaction utilizing creatine as a substrate. Creatine and creatine phosphate can be synthesized relatively easy and are believed to be non-toxic in mammals.

The use of creatine and analogues thereof for the treatment of diseases of the nervous system have already been described in WO-A-96/14063 AVICENA GROUP INC.

However, nowhere has the use of creatine kinase or creatine compounds for the treatment of bone and cartilage cells or tissues been specifically disclosed or advocated for the treatment of bone and cartilage in health and disease.

The present invention as claimed provides for new use of creatine kinase and creatine compounds (which modulate one or more of the structural or functional components of the creatine kinase /creatine phosphate system) as a therapeutic agents. More particularly, the present invention provides methods of:
a) treatment of bone or cartilage diseases (e.g. osteoporosis, osteoarthritis or periodontitis);
b) promoting growth or mineralisation of bone or cartilage cells and tissues;
c) conservative or operative treatments of bone fractures or bone defects;
d) applying bone or cartilage grafts to bone or cartilage fractures or defects;
e) tissue engineering by extracorporeal culture of bone or cartilage forming cells (obtained from a healthy individual or particular patient) in the presence of creatine to form a three-dimensional cell assembly which can be transferred in a subsequent step to a specific location having a bone or cartilage defect of the same particular patient;
f) metabolic engineering of bone and cartilage cells by transfection with DNA coding for creatine kinase in order to make said cells overexpress creatine kinase and thus together with creatine improve, stimulate and stabilize the physiological function of said cells and tissues for reimplantation into patients as outlined in section e).

In all these applications of creatine the essential function of creatine (Cr) is its ability to act as an energy source and regulator of cellular energy metabolism, as well as a cell protective agent against metabolic stress. In addition, creatine has surprisingly shown to exert a protective effect on early events of programmed cell death or apoptosis. These effects are all mediated by creatine kinase.

The surprising effect of the creatine compounds on bone and cartilage cells and tissues has been to speed-up time for and improve the process of healing wounds in bone or cartilage tissue caused by trauma or surgery, including bone fractures and the acceptance and bonding of artificial implants. The treatment with creatine compounds can be therapeutic for diseased patients, preventive for healthy people as well as geriatric for elderly people. A variety of creatine compounds may be used in connection with the invention, in particular those selected from the group of creatine, creatine phosphate, creatine pyruvate and cyclocreatine.

The creatine compounds may be in the form of a pharmacologically acceptable salt or combined with an adjuvant or other pharmaceutical agent effective to treat bone and/or cartilage cells. Compounds useful in the present invention are creatine compounds which modulate the creatine kinase system.

The present invention also provides pharmaceutical compositions containing creatine compounds in combination with a pharmaceutical acceptable carrier. Such carriers are disclosed e.g. in
"Principles of Tissue Engineering, Chapter 19: Biodegradable Polymers for Tissue Engineering, J.M.Pachence and J. Kohn, 1997, pag. 274-293; and in
"Der Orthopade, Bone replacement materials, J.M.Rueger, 2-1998, pag 73-79;

The compositions useful in the invention may be administered orally, in form of granulates or in a sustained release formulation. By sustained release is meant a formulation in which the composition becomes biologically available to the patient at a measured rate over a prolonged period. Such compositions are well known in the art.

The main route of Cr biosynthesis in mammals involves the formation of guanidinoacetate in the kidney, its transport through the blood and its methylation to Cr in the liver. Cr, exported from the liver and transported again through the blood, may then be taken up by the Cr-requiring tissues via the creatine transporter protein. When mammalian cells are cultured, creatine is available only in the amounts present in the serum added, which contains 0,05 - 0,10 mM Cr.
The enzyme creatine kinase (CK) plays a key role in the energy metabolism of cells that have intermittently high and fluctuating energy requirements. CK isoenzymes are found predominantly in skeletal and cardiac muscle, but also in spermatozoa (vertebrate and sea urchin sperm), electrocytes of the electric organ of electric fish, photoreceptor cells of the retina and the lens of the eye, brain (glial and neuronal cells of the cerebellum, glomerular structures of the cerebellum, neurones), the uterus and placenta, intestinal brush border epithelial cells and endothelial cells, kidney and rectal salt gland, adipose tissue, pancreas, thymus, thyroid and liver, cartilage and bone, macrophages, blood platelets, as well as in certain malignant tumours and cancer cells.
The reaction catalyzed by CKs, the reversible transfer of the phosphoryl group from phosphocreatine (PCr) to ADP, allows regeneration of the key cellular energy carrying molecule ATP. Cells contain a number of different CK isoforms, which are not evenly distributed in cells. They are compartmentalized in an isoform-specific fashion the two isoforms M-CK and B-CK are cytosolic, and two of the isoforms Mia-CK and Mib-CK are specifically mitochondrial. These various isoforms of CK are thought to constitute an intricate energy buffering and transport system, connecting sites of high energy phosphate production (by glycolysis and oxidative phosphorylation) to sites of energy consumption (ATPases).
The mitochondrial CK isoforms (Mi-CK) are located along the outer surface of the entire inner membrane and also at sites where the inner and outer membranes are in close proximity. At these latter sites, Mi-CK can directly use intra-mitochondrially produced ATP to generate PCr, which is exported to the cytosol where it serves as an easily diffusible, energy-storage metabolite. In contrast to the cytosolic CK isoforms, which are dimeric, Mi-CK, forms highly symmetrical, cube-like octamers (Schnyder et al. 1991) that can bind to the periphery of lipid membranes. Most importantly Mi-CK can mediate contact-site formation between the inner and the outer mitochondrial membranes and in addition, Mi-CK is functionally coupled to oxidative phosphorylation by the adenine nucleotide transporter which catalyzes ATP/ADP antiport across the inner membrane. Net PCr production can be stimulated by addition of extra-mitochondrial Cr, even in the presence of external ATP-regeneration systems and ATP sinks.

### Creatine and phosphocreatine in cartilage

Resting and hypertrophied cartilage both contain PCr. However, the distribution of PCr varies in the different zones of the cartilage. The highest content of Cr is in the resting cartilage. The other zones have similar amounts of Cr. On the other hand, the highest amount of PCr is found in the proliferative zone of cartilage with lower concentration in resting and hypertrophic cartilage. In calcified cartilage-bone, PCr is indetectable.
Experimental studies show that external addition of PCr promotes cartilage mineralization in organ and cell culture. The deposition of calcium in the cartilage matrix of the epiphysis of cultured embryonic chick femora is accelerated by the addition of very crude preparations of PCr and Cr at 0,1 mM in chick embryo extract with 20 % horse serum. Mineralization in differentiating chick limb bud mesenchymal cells in micromass cultures is promoted by the addition of 1 and 2 mM ATP or 2 mM PCr. The formed mineralized cartilage matrix is similar to that *in ovo.* The addition of ATP or PCr does not alter the rate of cell proliferation, the rate of matrix synthesis, the mean crystallite length or the rate of mineral deposition, when contrasted with cultures supplemented with inorganic phosphate. The ultrastructure of the cultured cells in the presence of 4 mM inorganic phosphate (Pi), 1 - 2 mM ATP or 2 mM PCr are similar at day 14 and 21. There are differentiated chondrocytes within the nodule containing hypertrophied and degenerating cartilage. At the edge of the nodule the cartilaginous matrix containing type II collagen, proteoglycans and matrix vesicles is surrounded by undifferentiated cells and type I collagen. ATP, PCr or Pi do increase the mineral to matrix ratio around the edge of the micromass but not in the centre of the cartilage nodule (low mineral to matrix ratio). There is no difference in the pattern of mineralization due to Pi, ATP or PCr.
Reduction of the Cr uptake by feeding rats with beta-guanidinopropionate (GPA) results in marked abnormalities in the epiphyseal growth plate of the rats. The zone of calcified cartilage is wider and extends into the metaphysis. The hypertrophic chondrocytes are vacuolated and poorly columnated and mineralization is less abundant and occurs also in the transverse septa. Vascular invasion is poor. There is a reduction in the osteoid formation. GPA interferes with the synthesis of pro-a type II and type X collagen in cultured chondrocytes.

### Creatine and phosphocreatine in bone

PCr increases the alkaline phosphatase (ALP) activity in SaOS-2 cells. The perichondral ossification in the diaphysis of cultured embryonic chick femora is accelerated by the addition of PCr and Cr preparations at 0.1 mM to chick embryo extract with 20% horse serum.

### Creatine kinase in cartilage

The level of CK activity is correlated to the chondrocyte maturation in the epiphysis and in the rib. There is an increase in CK activity from the resting-proliferative cartilage to the hypertrophic cartilage (6 fold) and the calcified cartilage-bone zone (17 fold). In resting and proliferating cartilage the predominant CK isoform is MM. M-CK is 1/3 to 1/5 of those in skeletal muscle (160'000 ng/mg protein) and the amount is independent of the age. In hypertrophic cartilage the MB-CK and BB-CK isoforms are predominant and B-CK is 30 - 47 fold higher than in skeletal muscle (60 ng/mg protein and B-CK shows a significant decrease with advancing age.
CK activity seems to be required for matrix synthesis and mineralization of the enchondral growth cartilage and chondrocytes in culture undergoing hypertrophy show an increase in the CK activity. CK activity peaks in the cartilage in rats of peripubertal age.
CK activity in the cartilage is stimulated by growth hormone (GH), by insuline-like growth factor 1 (IGF-I), by metabolite of vitamine D [24*R*,25(OH)₂D₃] in normal rats and in vitamin D-deficient rats, by PTH, by protease-resistant variants of parathyroide hormone (PTH), by 17b-estradiol in normal rats and in ovariectomized rats. Stimulation of BB-CK activity is followed by a parallel increase in DNA synthesis.
In rachitic cartilage the profile of CK is similar but the values in the hypertrophic and also in the calcified cartilage are lower than in the normal cartilage.

### Creatine kinase in bone

In embryonic chick bone there is BB-CK along with some MB and MM-CK activity. During early facial development there is a prominent anaerobic metabolism in the facial processes, BB-CK is present from the 9^{th} embryonic day, and during 10^{th} -15^{th} days MB-CK and MM-CK develop. The amount of bone produced during hetereotropic bone formation by implantation of BMP into muscle of rats shows an almost parallel relationship with the levels of S-100b protein, B-CK, hyaluronic acid and chondroitin sulphate and the activity of ALP. B-CK expression is modulated by transcriptional and posttranscriptional mechanisms during differentiation of osteoblastic cells. Enhanced activity of membrane pumps and changes in the cytoskeleton are related to the formation of extracellular matrix and mineralization.

In bone, similar to cartilage, BB-CK is also experimentally increased both *in vitro* and *in vivo* by IGF-I; by 1,25(OH)₂D₃; by PTH; by protease-resistant variants of PTH; by PGE₂; by 17b-estradiol (E₂). Furthermore, the stimulation of the bone cell energy metabolism by 17b-estradiol (E₂) and testosterone is sex specific, as shown in diaphyseal bone of weanling rats but not in epiphyseal cartilage. E₂ causes a 70-200% increase in CK activity *in vivo* and *in vitro* in ROS 17/2.8, in MC3T3-E1 cells and foetal rat calvaria cells and a 40% increase in rat epiphyseal cartilage cells. The stimulation of E₂ is dose- and time-dependent. Ovariectomized rats between 1-4 weeks after surgery show a stimulation of CK by E₂, 24h after injection. Both the basal and stimulated activity of CK is higher in the diaphysis and epiphysis than in the uterus. The effect of E₂ *in vivo* and in chondroblasts and osteoblasts *in vitro* is inhibited by high levels of the anti-oestrogen tamoxifen which by itself in high concentration shows stimulatory effects. Furthermore, gonadectomy causes a loss of the sex-specific response of diaphyseal bone to steroid hormones. CK activity peaks in diaphyseal bone and cartilage in rats of peripubertal age. Patients with autosomal dominant osteopetrosis Type II have an elevated level of BB-CK but patients with other sclerosing bone diseases do not show such an elevation in BB-CK.

For adult humans (70 kg) the daily dosage of chemically pure creatine monohydrate is in the range of 0,1 to 20,0 g per day, preferably with a loading phase of 4 times 4-6 g per day for the first 8-14 days and a maintenance dosage of 2-4 g per day for another 3 months with an interruption of the supplementation scheme for one month thereafter.
To improve bioavailability, chemically pure creatine monohydrate can be mixed with carbohydrates like maltodextrins and/or dextrose and others.

The various features of novelty which characterize the invention are pointed out with particularity in the claims annexed to and forming part of this disclosure. For the better understanding of the invention, its operating advantages and specific objects attained by its use, reference should be had to the accompanying drawings, examples and descriptive matter in which are illustrated and described preferred embodiments of the invention.

In the drawings:
Figure 1 is a graph showing Viability (NR) of monolayer osteoblast cell cultures at 1, 2 and 3 weeks in the absence (control) and presence of either 10 mM or 20 mM creatine in the medium. Least squares means ± 1,96 standard error;
Figure 2 is a graph showing metabolic activity (MTT) of monolayer osteoblast cell cultures at 1, 2, and 3 weeks in the absence (control) and presence of either 10 mM or 20 mM creatine in the medium. Least squares means ± 1,96 standard error;
Figure 3 is a graph showing mineralization of monolayer osteoblast cell culture at 2 and 3 weeks in the absence (control) and presence of either 10 mM or 20 mM creatine in the medium. Least squares means ± 1,96 standard error;
Figure 4 is a graph showing mineralization of micromass osteoblast cell culture at 2 and 3 weeks in the absence (control) and presence of either 10 mM or 20 mM creatine in the medium. Least squares means ± 1,96 standard error; and
Figure 5 is a graph showing embryonic rat femora wet weight after 3 weeks in organ culture, with and without 10 mM or 20 mM creatine. Least squared means ± 1,96 standard error.

### Aim of the experimental work

The effects of supplementation with creatine (Cr) and beta-guanidinopropionic acid (GPA; a Cr analogue and competitor of Cr uptake into the cell) on the differentiation of osteoblasts and chondrocytes *in vitro* were determined. The parameters investigated were viability (based on the physical uptake of neutral red and the metabolic activity), histochemical ALP activity and degree of mineralization, as well as the TEM ultrastructure.

### Methods

### Cell culture

This isolation technique is based on the ability of osteoblasts to migrate from bone onto a substratum. Parietal and frontal calvariae (4 per animal) were aseptically explanted from 6 day old IcoIbm rats. The calvariae were placed in Tyrode's balanced salt solution, calcium and magnesium free (TBSS). The periosteum was enzymatically removed with 0,05 % trypsin and 0,02 % collagenase A (0,76 U/mg) dissolved in TBSS (40 calvaria/20 ml). The calvariae were shaken for 70 minutes in a waterbath at 37°C. They were washed with TBSS and then transferred to 60 mm culture dishes (40 calvariae/dish) containing 5 ml of 0,02 % collagenase A (0,76 U/mg) in culture medium BGJ_{b} Fitton-Jackson modification and placed in the incubator for 4 hours. Afterwards the calvariae were washed with culture medium B supplemented with 10 % foetal calf serum (FCS). The calvariae were transferred into 60 mm culture dishes (4 frontal and 4 parietal/dish). The growth medium supplemented with 10 % FCS and 50 µg/ml ascorbate was completely changed every 48 h. The culture was kept for 3 weeks.
After 3 weeks the migrated cells were harvested. The dish was washed with TBSS and 5 ml of TBSS containing 0,05 % trypsin and 0,02 % collagenase A (0,76 U/mg) was added. After 1 hour in the incubator the dish was washed with culture medium BGJ_{b} supplemented with 10 % FCS. The dishes containing the calvariae and cells were rinsed with serum containing media BGJ_{b}. The cells obtained were filtered through a 40 µm nylon mesh to remove bone debris and cell aggregates. The suspended cells were centrifuged at 600 g for 5 minutes. The cell pellet was resuspended in serum containing medium BGJ_{b} and centrifuged. The viability of the resuspended cells was examined by the 'dye exclusion' of 0,4 % trypan blue and the cells counted in a haemocytometer. The inoculation densities were 2·10⁵ /10 cm² for monolayer and 2·10⁵/30 µl for micromasses. The micromass cultures were kept for 1 h in the incubator before 2 ml growth medium was added.

### Organ cultures

### Calvariae with periosteum

Parietal and frontal calvariae (4 per animal) were aseptically explanted from 6 day old IcoIbm rats. The calvariae were washed thoroughly with TBSS, then transferred into 60 mm culture dishes (4 frontal and 4 parietal/dish) containing growth medium BGJ_{b} supplemented with 50 µg/ml ascorbate either serum-free or with 10% FCS. The medium was changed completely every 48 h. The culture was kept for 3 weeks and then processed for histology.

### 'Denuded' calvariae

The periosteum was enzymatically removed with 0,05 % trypsin and 0,02 % collagenase A (0,76 U/mg) dissolved in TBSS (40 calvariae/20 ml). The calvariae were shaken for 70 minutes in a water bath at 37°C. They were washed with TBSS. The calvariae were then transferred to 60 mm culture dishes (40 calvariae/dish) containing 5 ml of 0,02 % collagenase A (0,76 U/mg) in culture medium BGJ_{b} and placed in the incubator for 4 hours. Afterwards the calvariae were washed with culture medium BGJ_{b} supplemented with 10 % FCS. The calvariae were transferred into 60 mm culture dishes (4 frontal and 4 parietal/dish). The growth medium BGJ_{b} supplemented with 50 µg/ml ascorbate was either used serum-free or with 10% FCS and was completely changed every 48 h. To study the effect of FCS the cultures were kept for 3 weeks and then processed for histology.

To study the bone regeneration capacity of calvariae they were kept as long-term cultures for 6, 9, 12, 15 weeks in growth medium with 10 % FCS. Every 3 weeks these calvariae were transferred into a fresh culture dish. At the endpoint the calvariae were processed for histology.

### Embryonic long bones

The rats were sacrificed on the 17^{th}-18^{th} day of pregnancy. The embryos were aseptically removed from the uterus and both femora were carefully dissected free into sterile TBSS under the stereo-microscope. Organ-culture of the rudiments was performed in 10 cm² plastic culture dishes. A Teflon carrier with a nylon mesh (20 µm pore size) was mounted in the dish, keeping the explants floating and ensuring optimal gas exchange and nutritional conditions. The right and the left femora from each animal were randomly assigned to the experimental or control group. The control groups were kept in 3 ml B with 50 µg/ml ascorbate. In the experimental group the growth medium was supplemented with either 10 mM Cr, 20 mM Cr, 1 mM GPA, 5 mM GPA, or 10 mM GPA. The growth medium was renewed every second day until day 10. Culture was carried out at 37,5 °C and in a 5 % CO₂ atmosphere. At 10 days the wet weight of each femora was determined on a microbalance. The result of each experimental femora was expressed relative to its collateral control. For the histological evaluation, the femora were fixed in 4% formaldehyde, dehydrated and embedded in methylmethacrylate. The 6 µm sections were stained by Pentachrome-Movat.

### Culture condition

All the cultures were kept at 37°C in a humidified atmosphere of 5 % CO₂, 95 % air.
All culture media were supplemented with 50 µg/ml ascorbate. To analyze the collagen types, 60 µg/ml beta-aminopropionitrile (beta-APN) was added to the culture medium. During cell isolation and inoculation no ascorbate was used to increase plating efficiency. No antibiotics and no beta-glycerophosphate were added. The media were completely changed every 48 hours (60 mm culture dish 5 ml ; 35 mm culture dish 2 ml).

### Morphology

### Alkaline phosphatase activity (histochemically)

The cells were histochemically stained for the alkaline phosphatase as described in the Sigma technical Bulletin no. 85L.

### Principle

Gently fixed cells were incubated in a solution containing naphtol AS-MX. As a result of phosphatase activity, naphtol AS-MX was liberated and immediately coupled with a diazonium salt forming an insoluble, blue pigment at sites of phosphatase activity.

### Solutions

- Fixative
   2 vol. Citrate buffer; dilute citrate concentrate 1:50
   3 vol. acetone
- Stain
   dissolve content of 1 capsule Fast Blue in 48 ml distilled water on a magnetic stirrer.
   add 2 ml of naphtol AS-MX solution just before use.

### Procedure

1. wash 3 x in TBSS
2. fixation 5 min. at 20°C
3. wash 3x with distilled water
4. stain 30' in the dark at room temperature (RT)
5. wash 3x with distilled water.

### Mineralization

### Principle

The most specific method for detecting calcified matrices is the von Kossa reaction. Silver staining indicates the presence of calcium phosphate aggregated with certain organic acids. Structural detail are completely obscured by the dark precipitate. Calcified tissue components are darkened in various shades from light brown to deep black, irrespective of their mineral content.

### Solution

- Silver nitrate
   5 % AgNO₃ in distilled water
- Pyrogallol
   1 % in distilled water
- Sodium thiosulphate
   1 % Na₂S₂O₃ 5 H₂O in distilled water

### Procedure

| | |
|---|---|
| 1. fixation in 4 % formaldehyde | 30 min. |
| 2. wash in distilled water | 3x |
| 3. silver nitrate | 30 min. in the dark |
| 4. wash with distilled water | 5x |
| 5. pyrogallol | 5 min. |
| 6. wash with distilled water | 5x |
| 7. sodium thiosulphate | 10 min. |
| 8. wash with distilled water | 5x |

### TEM preparation

### Principle

In an electron microscope the specimen is exposed to very high vacuum. Therefore, the tissue has to be fixed and stained with heavy metals to give contrast and only very dense material deflects electrons and forms images. The tissue is impregnated with heavy metals (uranium, lead) either before or/and after sectioning. Because electrons do not penetrate very deeply into the tissue, very thin sections (50-100 nm) have to be cut with either a glass or a diamond knife on an ultra microtome. For ultrathin sectioning the specimen has to be dehydrated and penetrated with monomeric resin which polymerizes.
For chemical fixation glutaraldehyde is mostly used. It crosslinks the proteins covalently to their neighbours. In order to stabilize the lipids, especially the cell membranes, osmium-tetroxide is used as a postfixation. To enhance the contrast the tissue is treated *en block* with uranyl acetate and the sections are subsequently stained with uranylacetate and lead citrate.

### Solutions

- 0,2 M Cacodylate buffer pH 7,4
   Stock A 25 ml
   Stock B 1,35 ml
   distilled water ad 100 ml
   Stock A 10,7 g Cacodylic acid sodium salt Trihydrate
   250 ml Distilled water
   Stock B 0,2 M HCl
- Fixation
   25 % glutaraldehyde (EM grade) 2 ml
   0,2 M cacodylate buffer pH 7,4 10 ml
   distilled water ad 20 ml
- Postfixation 1 % OsO₄ in 0.1 M cacodylate buffer pH 7,4
   1 vol. 2 % OsO₄
   1 vol. 0,2 M Cacodylate buffer pH 7,4
      2 % OsO₄
      fracture glass vial
      add distilled water
      sonicate 5 min.
      filter through 0,45 mm filter (Millex)
      keep in dark at 4°C
- 2 % aqueous uranyl acetate

### Procedure

| | |
|---|---|
| 1. Fixation at 20°C | 20 min. |
| 2. rinse in 0,1 M cacodylate buffer pH 7,4 | 3x 30 s |
| 3. post-fixation at 4°C | 1 h |
| 4. rinse in distilled water | 3x 30 s |
| 5. uranyl acetate at room temperature. | 1 h |
| 6. dehydration in a graded series of ethanol: | |
| 7. 70 %, 80 %, 90 %, 100 %, 100 %, 100 %. | every.5 min. |
| 8. LR White (Polysciences). | > 2 h |
| 9. Polymerization at 60°C. | overnight |

### Ultrathin sections

### Principle

Ultrathin sections were cut either with a glass knife or with a Drukker Diamond knife on a LKB III Microtome, placed on Formvar coated copper grids, and stained with heavy metals.

### Solutions

- 5 % uranyl acetate
1 g/20 ml.

- lead citrate according to Reynolds

| | |
|---|---|
| Pb(NO₃)₂ | 0,67 g |
| Sodium citrate | 0,88 g tri sodium citrate dihydrate |
| | 15 ml distilled water |

gentle shaking for 15 min.
add 4 ml 1 N NaOH, white precipitate dissolves
fill up to 25 ml distilled water
add distilled water to 25 ml
Filter both solutions through a Whatman No. 50 (hardened) before use.

### Procedure

All solutions were placed as drops on a parafilm. Individual grids were placed onto the droplets, to floating, section side down. Solid NaOH pellets were placed in a plastic dish in the same chamber to absorb CO₂ from the air to prevent carbon dioxide precipitation of lead salts. Both the staining solutions and the solid NaOH pellets were covered with a lid.

| | |
|---|---|
| 1. distilled water | |
| 2. 5 % uranyl acetate 10 min. | |
| 3. distilled water | 2x |
| 4. lead citrate | 10 min. |
| 5. M NaOH | 3x 30 s |
| 6. distilled water | 2x |
| 7. remove the remaining small amounts of water between the prongs of the forceps with filter paper and dry the grids on Whatman No. 50 filter paper with the section side up. When the grids were dry, they were placed in the storage box ready for use. | |

The sections were examined on a JEOL JEM 100 CX transmission electron microscope operated at 100kV. Micrographs were taken on Kodak EM 4303 film at standard magnifications of 2000, 5000, 20000 or 33000 times. Pictures were printed onto multigrade paper.

### Cell viability (MTT)

The Böhringer 'Cell Proliferation Kit I (MTT)' was used for the assay, but we used a different solvent to dissolve the MTT crystals.

### Principle

Originally, Mosmann, 1983 described the general principle involved in the detection of cell growth/cell death as indicated by the conversion of the tetrazolium salt (MTT) to the coloured formazan by mitochondrial dehydrogenases. The concentration of this can then be measured spectrophotometrically.

### Procedure

1. MTT Stock (5 mg/ml in sterile PBS) from Böhringer was diluted 110 with complete growth medium and sterile filtering.
2. The cells were incubated in 2 ml/10cm² MTT solution at 37°C for 3h.
3. The supernatant was carefully removed.
4. 4 ml/10cm² dimethylsulphoxide (DMSO) was added.
5. The dishes were placed on a shaker until the crystals were completely dissolved.
6. The absorbance of the supernatant (3 aliquots/dish) was read at 550 nm versus DMSO.

### Comments

If the absorbance was higher than 1, the samples were diluted with DMSO.

### Cell viability (neutral red, NR)

The method as described in (Lindl et al. 1994) was used.

### Principle

The uptake of NR into lysosomes is independent of the metabolic status of the cell.

### Solutions

- 0,5 mg Neutral red /ml growth medium, warmed up to 37°C for at least 2h, sterile filtering
- Extraction buffer
   50% ethanol in 1 % acetic acid

### Procedure

1. The cells were incubated in 2 ml/10cm² NR solution at 37°C for 3h.
2. The supernatant was removed,
3. washed with PBS, at least 3 times, until no crystals were present.
4. Addition of 4 ml/10cm² extraction buffer.
5. The absorbance of the supernatant (3 aliquots/dish) was read at 540 nm versus extraction buffer.
6. If the absorbance was higher than 1, the samples were diluted with extraction buffer.

### Statistics

The mean value and the standard deviation consisted of n independent experiments. The values for the individual experiments were gained from the mean of 3 aliquots of the same dish. To compare the treatment contrasts analysis of variance models were evaluated.

In experiments carried out as paired designs a model accounting for the animals considered as blocks were examined. Main effects and interaction effects were examined by F-Tests.

'Least Squares Means' were calculated to yield average means accounted for the other variables in the model. LS Means were compared by using Tukey's multiple range test.
QQ-Plots of the residuals and Tukey-Anscombe plots (residuals x predicted) were analyzed to check for normal distribution assumption.

### Results

### Monolayer Cell culture

### Cell viability and metabolic activity

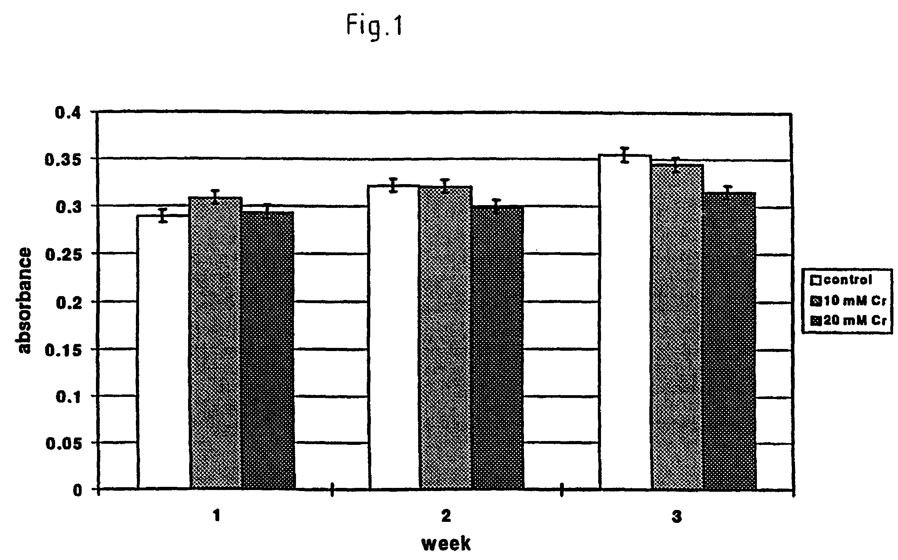

With respect to cell viability, in all groups, neutral red (NR) stained mainly the cells at the edge and the top of the nodules as well as the cells between them. Staining with trypan blue showed that the cells/matrix between the nodules and the nodules themselves were stained.
Preliminary quantitative data on the NR uptake showed that the Cr and the GPA groups had similar results as the control group at 2 weeks. Concerning the metabolic activity measured by the MTT reaction, the Cr groups were slightly stimulated when compared to the control, but the 5 M or the 10 mM GPA had lower values than the control, indicating some inhibition of the GPA at these particular concentration. The 1 mM GPA group was similar to the control. At 3 weeks, all experimental groups had a lower NR uptake than the control. The Cr stimulated the MTT reaction, and the 1 mM or 5 mM GPA had lower values than the control. The 10 mM GPA was comparable to the control. These results were indicating that the Cr had a stimulatory effect on the metabolism of the cells and the GPA had some inhibition on the mitochondrial activity of the cells.
In the further experiments to quantify the viability and the metabolic activity of the cells, the creatine groups were only used. Statistical analysis of the NR uptake (Fig. 1) showed that there was a small but significant interaction effect (p<0.05). This meant that the effect of treatment with creatine was not similar at the different time points. The NR uptake of the control group at 1 week was significantly lower than that of 2 and 3 week (p<0,03, respectively p<0,0002). The NR uptake of the 10 mM Cr group was significantly higher at 3 weeks as compared to that at 1 week (p<0,02). At 1 and 2 weeks there was no significant difference between the groups. At 3 weeks the control group was significantly (p<0,008) higher than the 20 mM Cr group. The increase in the NR uptake of the control group during the culture indicated that there was an increase in the cell number. The difference of the control group and the 10 mM Cr was no-significant. This showed that there was no toxic effect of the creatine at this particular concentration. This was in contrast to the 20 mM Cr, which had an significantly lowered NR uptake when compared to the control group. This indicated some toxic effects on the proliferation of the cells.

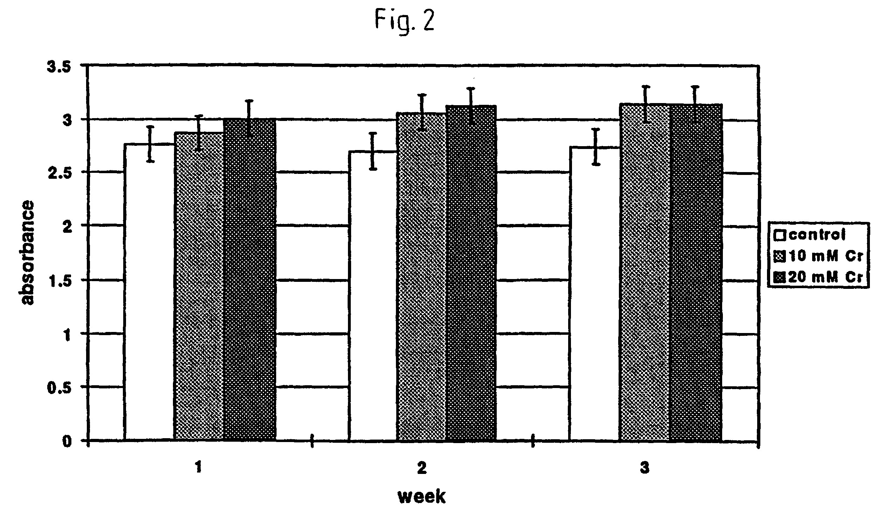

Concerning the metabolic activity (MTT) of the cells (Fig. 2), creatine had an effect on osteoblasts in culture. At 1 week all groups were similar. At 2 weeks the control group was significantly lower than the 10 mM Cr and the 20 mM Cr (p<0,015, respectively p<0,0025). At 3 weeks both the 10 mM Cr and 20 mM Cr were significantly higher than the control group (p<0,001). These data showed that in general creatine stimulated the metabolic activity of osteoblasts from the second week onwards.

### Morphology

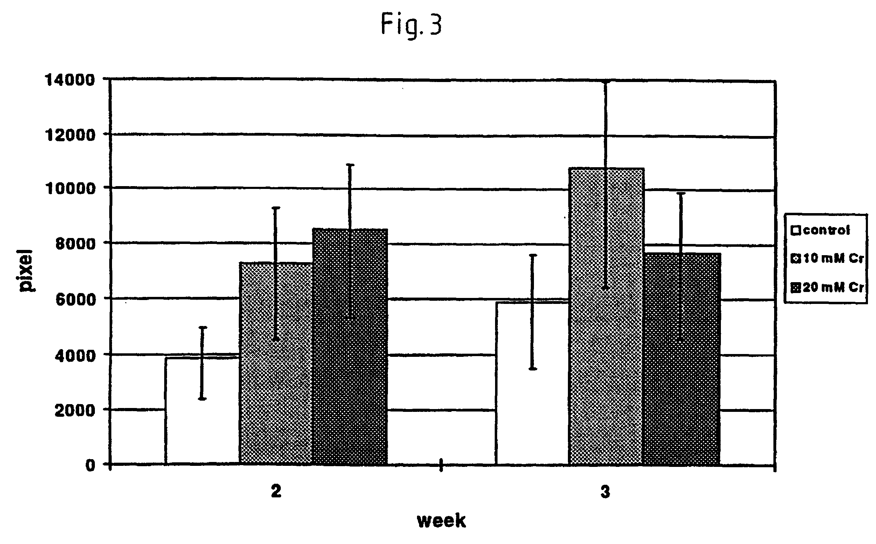

After 1 week the cells in all groups formed a monolayer with ALP positive cells. Some cells had a really high ALP activity. After 2 weeks all groups formed some small mineralized nodules. After 3 weeks the overall staining for ALP activity was similar in all groups. At higher magnification the GPA groups showed a different staining pattern for the ALP activity compared with the control and the Cr groups. The cell density around the nodules was lower than in the control and the Cr groups. At 3 weeks the mineralized nodules increased in size and number compared with 2 weeks. All the experimental groups showed a higher mineralization than the control group. The calcification pattern of the GPA groups was different from the control and the Cr groups in such that the mineralization was not limited to the nodules and more single cells showed calcification than the control and Cr groups.
In the further experiments to quantify the calcification by image analysis of a center area (123 mm²) of the culture dish, only the creatine groups were compared to the control groups, with the GPA treated cells not further evaluated. Statistical analysis showed that the calcified area in the 20 mM Cr group (Fig. 3) was significantly higher than the one in the control group (p<0,02) at 2 weeks. At 3 weeks, 10 mM Cr group had more mineralization than the control, whereas the 20 mM Cr was less effective, but there was no significant difference between the various groups.

### TEM-Monolayer

The ultrastructure of the control group at 1 % FCS was similar to the cells kept at 10 % FCS.
The ultrastructure showed that there were no obvious differences between the control, the 10 mM Cr group, the 20 mM Cr group the 1 mM GPA group, and the 5 mM GPA group.
In all groups there was collagen production and mineralization. The cytoplasm of cells had the typical features of osteoblasts such as a well developed rER, Golgi area, mitochondria, vesicles, micro filaments. The cells had many cell processes which were in close contact to each other. There was abundant collagen production. The collagen fibrils were seen in membrane folds. The diameter of the fibrils was rather uniform. In the area of mineralization, the individual fibrils seem to coalesce into larger units. The mineralization pattern was similar in all groups. There were high density needle-like structures at the lowest cell layers. At the mineralization front the same material was observed around collagen fibrils and in close apposition to the plasma membranes. Mineralized patches were seen in the collagenous matrix. In areas with high calcification the details of the matrix were no longer visible.

### Micromass cell culture

The NR uptake was similar in all groups at 1 and 2 weeks. At 3 weeks the 20 mM Cr groups had a significantly lower NR uptake (p<0,005, respectively p<0,003) than the control and the 10 mM Cr group.

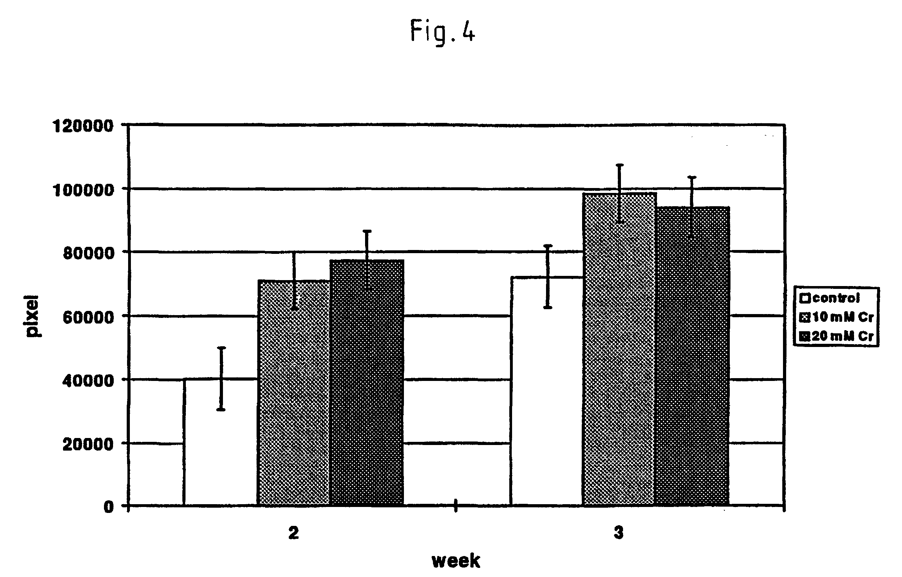

The mitochondrial activity (MTT conversion) was similar in all groups at 1,2, and 3 weeks. However, the Cr groups at 10 mM and 20 mM concentration had a significantly higher MTT reaction at 2 weeks than at 1 week (p<0,02, respectively p< 0,006). At 3 weeks, the 20 mM Cr had a significantly lower MTT conversion than at 2 weeks (p<0,015).
Concerning the mineralized area (Fig. 4), the creatine groups at 10 mM and 20 mM concentrations had significantly more mineralization (p<0,00025) than the control at 2 weeks. At 3 weeks the mineralized area was significantly higher in the creatine groups at 10 mM and 20 mM concentrations than in the control (p<0,0035, respectively p<0,03). Furthermore, the control and the 10 mM Cr groups showed a significantly higher mineralization at 3 weeks than at 2 weeks (p<0,0005, respectively p<0,0015).

### Organ Culture

### Femora

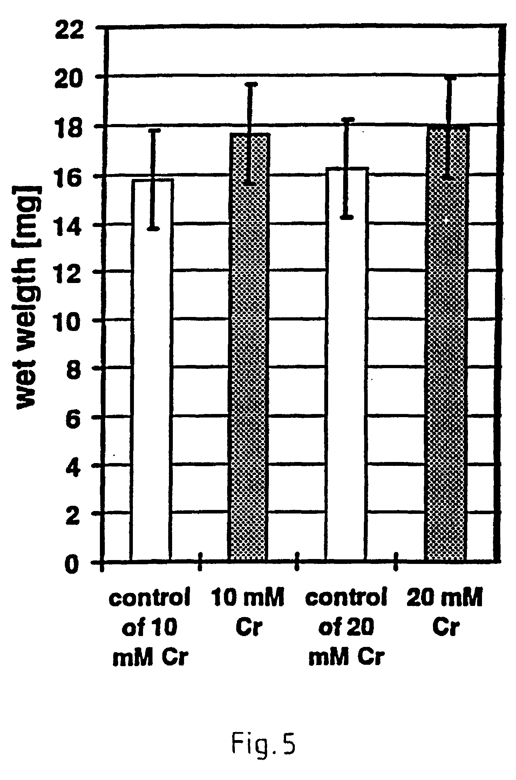

The control (Fig. 5) had significantly lower wet weights than 10 mM Cr (p<0,005), 20 mM Cr (p<0,001), 5 mM GPA (p<0,0005) and 10 mM GPA (p<0,015). The results of 1 mM GPA were not significantly different from the control.

### Assessment of the experimental data

There was a small but significant interaction effect of creatine in the NR uptake in monolayer cultures. This meant that the effect of the treatment with creatine was not similar at the different time points. In the control group there was a significant increase in the NR uptake during the culture. This was due to an increase in the cell number. At 1 and 2 weeks there was no significant difference between the groups. However, the effect of the 10 mM Cr on the NR uptake was significant at 3 weeks compared to that at 1 week. At 3 weeks the 20 mM Cr group was significantly lower than the control group. This indicated some toxic effects, which resulted in a reduced proliferation of the cells. This was not observed in the 10 mM Cr group, which was similar to the control group. This showed that there was no toxic effect of the creatine at this particular concentration of 10 mM. In the micromass cultures, the NR uptake was similar in all groups at 1 and 2 weeks. However at 3 weeks the 20 mM Cr had significantly less than the control and the 10 mM Cr. In contrast to the monolayer cultures, the NR uptake was not reduced during culture. This could be explained by the fact that in micromass cultures, the cells were migrating off the initially inoculated drop of cells and so the cell number is slowly increasing.
The results concerning the metabolic activity of monolayer cultures osteoblasts showed a significant stimulation of these cells by creatine at both concentrations, 10 mM and 20 mM, from the second week onwards. In the micromass cultures, the increase in the MTT conversion was only significant in the Cr groups at 2 weeks compared to the one 1 week. This indicated that the micromass culture behave differently than the monolayer cultures. This was not astonishing because in the micromass cultures, the cells have a very early cell-cell contact and so the differentiation process started earlier than in the monolayer cultures where the cells have first to proliferate to make cell-cell contacts. Nevertheless, the Cr significantly stimulated the metabolic activity of the micromass cultures at the early mineralization at 2 weeks when compared at 1 week.
In all groups, NR stained mainly the cells at the edge and the top of the nodules and between them. Staining with trypan blue in all groups showed that the cells at the bottom of the culture dish stained as well as those in the nodules. This could either be attributed to an artefact of staining or it might be that the cell membrane of the stained cells was really damaged. Concerning the artefact possibility, trypan blue would also stain extracellular proteins. An indication of the presence of damaged cell membranes was obtained from the TEM ultrastructure studies of monolayer cultures. Some of the cells near the culture dish surface had electron dense, needle-like material in the cytoplasm. It could be that the lower cells of the mineralizing nodule did not get enough nutrition or oxygen by diffusion through all other cell layers. It is very important that the cells stay alive because only viable cells can regulate mineral deposition and prevent dystrophic calcification. The presence of dead cells can lead to an increased mineralization.
After 2 weeks all groups formed some small mineralized nodules which increased in size and number after 3 weeks. Calcification was also observed in single cells. Mean values were higher in the Cr groups than the control after 2 and 3 weeks. In the micromass cultures, the Cr groups had significantly more mineralization than the controls.

Thus, Cr enhanced the formation of mineralized nodules by increasing the metabolic activity of the osteoblasts in cultures. It is suggested that there is an elevation in PCr turnover during tissue mineralization because the creatine phosphate concentration in calcified cartilage is low and the activity of the kinase in this zone is high. Furthermore, the energy metabolism in cartilage may affect the morphogenic events of skeletal growth.
There is evidence that mineralizing cells require a large amount of energy. Differentiating osteogenic cells have mitochondria with condensed cristae that represent an increased rate of energy metabolism. These mitochondria are particularly abundant in the differentiation stage and decline as the culture matures. Mineralization is thought to be associated with an optimal level of energy metabolism rather than extreme hypo- or hyperoxia.
Increased glycolysis with constant mitochondrial activity results in an augmented energy metabolism and increased ATP production. This increased availability of ATP could be a reason why osteoblasts synthesize more collagen when they are exposed to a high pH. An increased cell differentiation, during the formation of bone and cartilage, is accompanied by enhanced activities of ATPase and lactate, malate and glucose-6-phosphate dehydrogenases. Maximum activity is observed at the onset of the matrix deposition, followed by a decrease of enzyme activities during the transformation of osteoblasts to mature osteocytes and at the hypertrophy of chondrocytes. Histochemical ATPase activity, detected in osteoblasts, parallels the metabolic activity and viability of these cells. The ATPase activity in bone and cartilage cells is far less than in skeletal muscle, blood vessels and bone marrow. Osteoclasts reveal strong ATPase activity followed in intensity by osteoblasts, osteochondrogenic cells and lastly osteocytes. Cartilage cell activity, determined in this way, is generally weaker than osteoblastic activity. Young cell compartments reveal greater activity than those of older animals, with peak activity usually observed to 5 weeks of age. With increasing age and reduced functional demands the ATPase activity diminishes except in articular cartilage cells.
Inhibition of the glycolysis causes both a reduction in collagen synthesis and a hypermineralization in tibiae of chick embryos over a wide range of [Ca x Pi] in the medium (Pi 0,5 mM - 3,0 mM and 1,8 mM Ca²⁺). Furthermore, in the absence of glutamine there is more cell necrosis. Glutamine enters the citric acid cycle at a-ketoglutarate and provides biosynthetic precursors and NADH. NADH enters the oxidative phosphorylation and provides ATP. Inhibition of the activity of NAD-dependent enzymes associated with the production of ATP impairs cartilage formation resulting in limb shortening.
GPA, a competitive inhibitor of Cr entry into cells, seems to have adverse effects on both the metabolism and the viability of the cells, but mineralization is increased. This could be explained by the fact that cell death can also lead to mineralization. Since metabolic activity of creatine treated cells was generally higher compared to controls, the same parameter was lower in GPA, it was concluded that increased mineralization in the Cr treated groups was due to the metabolic stimulation of osteoblasts, whereas the one in GPA treated cells was mainly due to cell death. It is shown that growth plate cartilage cannot adapt to the metabolic stress imposed by GPA administration resulting in a disturbed enchondral bone formation in vivo and in vitro. The zone of calcified cartilage is wider and extends into the metaphysis. The hypertrophic chondrocytes are vacuolated and poorly columnated and mineralization is less abundant and occurs also in the transverse septa. Vascular invasion of the tissue is poor. There is a reduction in the osteoid formation. GPA interferes with the synthesis of pro-a type II and type X collagen in cultured chondrocytes. In long-term shell-less culture in the presence of GPA, the total CK activity is not altered but the CK isoenzyme profile is disturbed. The activity of BB-CK is suppressed in the long bones, but the isoenzyme distribution of calvariae is not affected. Normal embryonic cartilage contains nearly equal proportions of MM-CK and BB-CK. Embryonic calvariae and bone mainly express BB-CK. Feeding of rat and mice with GPA progressively decreases the concentrations of Cr and PCr in heart and skeletal muscle and leads to marked morphological changes mainly affecting mitochondria. A population of enlarged, rod-shaped mitochondria with characteristic crystalline intramitochondrial inclusions appears in adult rat cardiomyocytes in vitro. This phenomenon is fully reversible if the cell culture medium is supplemented with Cr. The appearance of highly ordered intra-mitochondrial inclusions correlates with a low intracellular total Cr content. Immunofluorescence and immuno-electron microscopy shows that these inclusions are enriched for Mi-CK. In the GPA treated osteoblasts, the mitochondria were similar to the control and Cr groups. So osteoblasts respond differently to GPA than do muscle cells. It is shown that GPA had comparably less influence on the Cr and PCr contents of brain. Soleus mitochondria show a 4 fold increase in Mi-CK protein and a 3 fold increase in adenine nucleotide translocator protein compared to the control.

### Conclusions

Creatine stimulates via the action of creatine kinase and other enzymes regulated by creatine or phosphocreatine, like AMP-dependent protein kinase the mineralization of osteoblasts in culture by increasing the metabolic activity of the cells in monolayer culture. In micromass cultures the creatine enhanced the mineralization but the metabolic activity was similar to the control. However, at 2 weeks the MTT conversion was significantly increased in the creatine group when compared to 1 week. Creatine had probably some effects on the differentiation process of the cells in this cell culture model. During nodule formation and subsequent calcification, the cells need a large amount of chemical energy. Biosynthesis of matrix collagen and proteoglycans, and/or the proliferation of the cells are increased. Creatine, as an external energy supply, has the advantage that it does not decrease the pH in the growth medium, thus avoids an inhibition of glycolysis and collagen synthesis.

Creatine also increases the wet weight of embryonic femora (Fig. 5) in organ culture. Indicating that not only bone but also cartilage cells benefit from external creatine supply. The biosynthesis of the matrix collagen and proteoglycan, and/or the proliferation of the cells are stimulated.

Creatine can therefore be applied as a food additive or supplement for humans and animals to support the recovery after trauma and orthopaedic surgery of fractures and bone defects.

Creatine has also potential to stimulate the metabolism of osteoblasts in patients suffering from osteoporosis.
Also the treatment of degenerative cartilage diseases, such as arthritis is supported by creatine.

The treatment of large bone defects is still a demanding task for surgeons. Patients suffering from large bone defects can be treated with bone grafting from the illiac crest to the defect or by applying callus distraction or segment transport. All these procedures are very painful for the patient and additionally the amount of bone graft is limited. The use of tissue engineering offers a solution to this problem. Bone forming cells (osteoblasts, mesenchymal stem cells, periosteal cells, stromal bone marrow cells or satellite cells of the muscle) as well as chondroblasts of healthy individuals or from a patient himself are cultured as monolayers, micromass cultures or in a three-dimensional, biodegradable scaffold in the presence of creatine. At a later point in time, the bone or cartilage cells or cell-seeded sponges, foams or membranes will be transferred to the defect in the patient. The most critical step in this approach is the cell culture work. It is fundamental that the cells survive, proliferate and differentiate *in vitro*. Therefore, culture conditions need to be optimal. In this respect, addition of creatine to the culture medium as a supplement is beneficial.

Although bone and cartilage cells express creatine kinase, albeit at relatively low levels compared to muscle and brain cells, it is surprising that over- expression of creatine kinase together with creatine supplementation improved proliferation, metabolic stability and resistance towards different stressors, e.g. toxins, heat, metabolic overload etc. of cartilage and bone cells. Thus bone forming cells (osteoblasts, periostal cells, stromal bone marrow cells or satellite cells of muscle) and cartilage forming cells (chondroblasts) removed from healthy individuals or from a patient to be treated are brought into cell culture and transfected with complementary DNA coding for creatine kinase isoforms (either cytosolic muscle-type MM-CK, cytosolic ubiquitous brain-type BB-CK or the heterodimeric MB-CK hybride enzyme, or sarcomeric- or ubiquitous mitochondrial Mi-CK's or combinations thereof). Complementary DNA (cDNA) can be obtained by reverse transcribing (RT) mRNA of CKisoenzymes, by RT-polymerase-chain reaction (RT-PCR), or by other methods using the appropriate primers corresponding to the respective CKisoenzymes.

The methods of gene transfer for cDNA's encoding for creatine kinase isoforms will encompass the entire selection of possible transfection techniques, as well as new techniques developed and made accessible to the public domain in the future, such as transfection via microinjection of cells, microsphere bombardment or DNA-precipitate transfection, as well as transfection via various viruses, viral and non-viral vectors or plasmids (single copy- and multi-copy plasmids), cosmids or artificial chromosomes. Creatine kinase expression is made under the control of weak or strong tissue specific promotors. Built-in selection markers, e.g. resistance towards antibiotics, toxins or others, make it possible to select for transfected cells which then are expanded in culture as described above in the presence of 1 to 20 mM creatine.

Cartilage or bone cells transfected with creatine kinase cDNA, made to overexpress creatine kinase isoenzyme(s) are then selected on a selection medium and expanded and cultivated either as monolayers, micromass cultures or on three-dimensional, biodegradable scaffolds or tissue sponges (as described above) to form in vitro genetically engineered cartilage- and bone pre-tissues which can be transplanted into the areas of cartilage and/or bone defects. For example, such transfected cartilage cells can be injected into arthritic joints to repopulate the areas of defect and repair chondro-degenerative defects in this joint by proliferation and producing new chondrocyte-derived extracellular matrix. Similarly, transfected bone-forming cells can be reimplanted into areas of bone defect to initiate regeneration and growth of bone mass in patients.

Since creatine kinase and creatine/phosphocreatine play an important role in the generation and maintenance of cartilage- and bone tissues, such tissues, genetically engineered to overexpress creatine kinases and being supplemented by externally added creatine or creatine analogues, are growing better after transplantation into areas of cartilage and/or bone defect in patients supplemented orally and/or locally with creatine.
Genetic engineering of creatine kinase into cartilage and bone cells in conjunction with creatine supplementation improves the proliferation, growth and specific function of these cells, e.g. the formation of extracellular cartilage- or bone-specific matrix. This metabolic engineering procedure followed by creatine supplementation is beneficial for cartilage and bone formation, healing and repair, as well as for mineralization.

The concentration of the creatine compound in the culture medium should preferably be in the range of 10 - 20 mM. The culture medium typically contains 0,1 % to 5,0 %, preferably 0,5 % to 2 % foetal calf serum. Furthermore the culture medium should contain 10 to 250 µg, preferably 25 to 100 µg ascorbic acid or an equivalent amount of a pharmaceutically acceptable ascorbate. The cell culture is started with 2'000 to 100'000 cells, preferably 10'000 to 50'000 cells.

The creatine compound can be administered in combination with hormones, preferably selected from the group of parathyroid hormone-related protein, thyroid hormone, insulin, sex steroids (estrogen, androgen, testosterone), prostaglandins, or glucocorticoids.

Further, the creatine compound can be administered in combination with intermittent administration of parathyroid hormone, preferably in combination with 1,25(OH)₂ vitamin D₃ and analogues or metabolites of vitamine D, calcitonine, estrogen, or bisphosphonates.

The creatine compound can be administered in combination with vitamins, preferably selected from the group of 1,25(OH)₂ vitamin D₃ and analogues or metabolites of vitamine D, of vitamin C/ascorbate or of retinoids.

The creatine compound can also be administered in combination with growth factors, preferably selected from the group of insulin like growth factors (IGF), transforming growth factor b family (TGF-b), bone morphogenic proteins (BMP), basic fibroblastic growth factor (bFGF), platelet derived growth factor (PDGF), or epidermal growth factor (EGF).

The creatine compound can be also administered in combination with cytokines, preferably selected from the group of interleukins (IL), interferons, or leukaemia inhibitory factor (LIF).

The creatine compound can be also administred in combination with matrix proteins, preferably selected from the group of collagens, glycoproteins, hyaluronan, or proteoglycans.
As glycoproteins those selected form the group of:
a) alkaline phosphatase,
b) osteonectin (ON),
c) gamma-carboxy glutamic acid-containing proteins, preferably matrix gla protein or osteocalcin or bone gla protein (OC),
d) arginine-glycine-asparagine-containing proteins, preferably thromspondin, fibronectin, vitronectin, fibrillin, osteoadherin, sialoproteins (osteopontin or bone sialoprotein BSP)
have shown a particular usefulness.
As proteoglycans those selected form the group of:
a) aggrecan,
b) versican,
c) biglycan,
d) decorin
have shown a particular usefulness.

The creatine compound can be administered in combination with serum proteins, preferably selected from the group of albumin or alpha-2HS glycoprotein.

The creatine compound can be also administered in combination with enzymes, preferably selected from the group of metalloproteinases, collagenases, gelatinases, stromelysins, plasminogen activators, cysteine proteinases or aspartic proteinases.

The creatine compound can be also administered in combination with calcium salts, bone meal or hydroxyapatite.

The creatine compound can be also administered in combination with fluoride salts, preferably sodium fluoride or monosodium fluorophosphate.

The creatine compound can be also administered in combination with peptides, preferably selected from the group of amylin, vasoactive agents or neuropeptides.

The creatine compound can be also administered in combination with antioxidants, preferably selected from the group of cysteine, N-acetyl-cysteine, glutathions or vitamins A, C, D or E.

The creatine compound can be also administered in combination with a substance selected from transferrin, selenium, boron, silicon, or nitric oxide.

In a preferred embodiment of the invention the agent is essentially free of dihydrotriazine. It has been found that dihydrotriazine is a toxic impurity of commercially available creatine and that it has an adverse effect for the patient.
For the same reason the agent should be essentially free of dicyano-diamide which is also toxic impurity of commercially available creatine.
It is further advantageous to an agent which is essentially free of creatinine as a natural degradation product of creatine.

The agent according to the invention can be administered to a human patient preferably in an amount of 1,4 to 285 mg per day.

In a preferred embodiment of the invention said creatine compound is selected from the group of creatine, creatine. phosphate, creatine pyruvate, cyclocreatine, homocreatine or homocyclocreatine.

In a further preferred embodiment of the invention said creatine analogue has the general formula:

Z₁===C(===Z₂)===X-A-Y

and pharmaceutically acceptable salts thereof, wherein:
(a) Y is selected from the group consisting of: -CO₂H, -NHOH, -NO₂, -SO₃H, -C(=O)NHSO₂J and -P(=O) (OH) (OJ), wherein J is selected from the group consisting of: hydrogen, C₁-C₆ straight chain alkyl, C₃-C₆ branched alkyl, C₂-C₆ straight alkenyl, C₃-C₆ branched alkenyl and aryl;
(b) A is selected from the group consisting of: C, CH, C₁-C₅ alkyl, C₂-C₅ alkenyl, C₂-C₅ alkynyl, and C₁-C₅ alkoyl chain, each having 0-2 substituents which are selected independently from the group consisting of:
   (1) K, where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, C₄-C₆ branched alkoyl, K having 0-2 substituents independently selected from the group consisting of: bromo, chloro, epoxy and acetoxy;
   (2) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy; and
   (3) -NH-M, wherein M is selected from the group consisting of: hydrogen, C₁-C₄ alkyl, C₂-C₄ alkenyl, C₁-C₄ alkoyl, C₃-C₄ branched alkyl, C₃-C₄ branched alkenyl, and C₄-C₆ branched alkoyl;
(c) X is selected from the group consisting of: NR₁, CHR₁, CR₁, O and S, wherein R₁ is selected from the group consisting of
   (1) hydrogen;
   (2) K where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
   (3) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
   (4) a C₅-C₉ Alpha-amino-omega-methyl-omega-adenosylcarboxylic acid attached via the omega-methyl carbon;
   (5) a C₅-C₉ Alpha-amino-omega-aza-omega-methyl-omega -adenosylcarboxylic acid attached via the omega-methyl carbon; and
   (6) a C₅-C₉ Alpha-amino-omega-thia-omega-methyl-omega-adenosylcarboxylic acid wherein A and X are connected by a single or double bond;
(d) Z₁ and Z₂ are chosen independently from the group consisting of.: =O, -NHR₂, -CH₂R₂, -NR₂OH; wherein, Z₁ and Z₂ may not both be =O and wherein R₂ is selected from the group consisting of:
   (1) hydrogen;
   (2) K, where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of bromo, chloro, epoxy and acetoxy;
   (3) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
   (4) a C₄-C₈ Alpha-amino-carboxylic acid attached via the omega-carbon;
   (5) B, wherein B is selected from the group consisting of: -CO₂H, -NHOH, -NO₂, -SO₃H, -C(=O)NHSO₂J and -P(=O) (OH) (OJ), wherein J is selected from the group consisting of: hydrogen C₁-C₆ straight alkyl, C₃-C₆ branched alkyl, C₂-C₆ straight alkenyl, C₃-C₆ branched alkenyl and aryl; wherein B is optionally connected to the nitrogen via a linker selected from the group consisting of: C₁-C₂ alkyl, C₂ alkenyl, and C₁-C₂ alkoyl ;
   (6) -D-E, wherein D is selected from the group consisting of: C₁-C₃ straight chain alkyl, C₃ branched alkyl, C₂-C₃ straight alkenyl, C₃ branched alkenyl, C₁-C₃ straight alkoyl, and aryl; and E is selected from the group consisting of: -(PO₃)ₙNMP, where n is 0-2 and NMP is a ribonucleotide monophosphate connected via the 5'-phosphate, 3'-phosphate or the aromatic ring of the base; -[P(=O)(OCH₃)(O)]ₘ-Q, wherein m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose of the aromatic ring of the base; and an aryl group containing 0-3 substituents chosen independently from the group consisting of: Cl, Br, epoxy, acetoxy, -OG, -C(=O)G, and -CO₂G, where G is independently selected from the group consisting of: C₁-C₆ straight alkyl, C₂-C₆ straight alkenyl, C₁-C₆ straight alkoyl, C₃-C₆ branched alkyl, C₁-C₆ branched alkenyl, C₄-C₆ branched alkoyl; wherein E may be attached at any point to D, and if D is alkyl or alkenyl, D may be connected at either or both ends by an amide linkage; and
   (7) -E, wherein E is selected from the group consisting of: -(PO₃)ₙNMP, where n is 0-2 and NMP is a ribonucleotide monophosphate connected via the 5'-phosphate, 3'-phosphate or the aromatic ring of the base; -P(P(=O)(OCH₃)(O))ₘ-Q, wherein m is 0-3 and Q is a ribonucleoside connected via the ribose or the aromatic ring of the base; -[P(=O)(OH)(CH₂)]ₘ-Q, wherein m is 0-3 and Q is a ribonucleoside connected via the ribose of the aromatic ring of the base; and an aryl group containing 0-3 substituents chosen independently from the group consisting of: Cl, Br, epoxy, acetoxy, -OG, -C(=O)G, and -CO₂G, where G is independently selected from the group consisting of: C₁-C₆ straight alkyl, C₂-C₆ straight alkenyl, C₁-C₆ straight alkoyl; C₃-C₆ branched alkyl, C₃-C₆ branched alkenyl, C₄-C₆ branched alkoyl; and if E is aryl, E may be connected by an amide linkage;
(e) if R₁ and at least one R₂ group are present, R₁ may be connected by a single or double bond to an R₂ group to form a cycle of 5 to 7 members;
(f) is two R₂ groups are present, they may be connected by a single or double bond to form a cycle of 5 to 7 members; and
(g) if R₁ is present and Z₁ or Z₂ is selected from the group consisting of -NHR₂, -CH₂R₂ and -NR₂OH, then R₁ may be connected by a single or double bond to the carbon or nitrogen of either Z₁ or Z₂ to form a cycle of 4 to 7 members.

The various modifications and preferred embodiments characterized in the dependent claims have produced a stimulatory effect on bone and/or cartilage.

While the foregoing description and drawings represent the preferred embodiments of the present invention, it will be obvious for those skilled in the art that various changes and modifications may be made therein without departing from the scope of the present invention.

## Claims

1. The use of creatine compounds, including analogues or pharmaceutically acceptable salts thereof, for the in vitro treatment of bone cells, cartilage cells or mesenchymal stem cells and tissues for transplantation into an animal or human.

2. The use of creatine compounds, including analogues or pharmaceutically acceptable salts thereof, for the preparation of an agent for the in vivo treatment of bone or cartilage diseases.

3. Use according to claim 2, **characterized in that** said creatine compound or said agent is used for the therapeutic treatment of bone or cartilage diseases, preferably of osteoporosis, osteoarthritis or periodontitis, or for the prophylactic treatment of said bone or cartilage diseases.

4. Use according to claim 1 or 2, **characterized in that** said creatine compound or said agent is used for promoting growth and mineralisation of bone or cartilage cells and tissues.

5. Use according to claim 1 or 2, **characterized in that** said creatine compound or said agent is used for conservative or operative treatments of bone fractures or bone defects.

6. Use according to claim 5, **characterized in that** said creatine compound or said agent is incorporated in a bone or cartilage graft to be applied to said fractures or defects.

7. Use according to claim 5, **characterized in that** said creatine compound or said agent is incorporated in three dimensional constructs of osteoblasts, chondrocytes or mesenchymal stem cells designed for tissue engineering of said bone or cartilage defects.

8. Use according to claim 1, **characterized in that**, bone or cartilage forming cells obtained from a healthy individual or a particular patient are cultured in the presence of said agent to form a three-dimensional cell assembly which is transferrable in a subsequent step to a specific location having a bone or cartilage defect of the same particular patient.

9. Use according to claim 2, **characterized in that** said agent is used for improving acceptance and osseous integration of bone implants.

10. Use according to claim 9, **characterized in that** said bone implants are endoprosthesis, preferably joint endoprosthesis.

11. Use according to one of the claims 1 to 10, **characterized in that** said creatine compound or said agent is selected from the group of creatine, creatine phosphate, creatine pyruvate, cyclocreatine, homocreatine or homocyclocreatine.

12. Use according to one of the claims 2 - 7 or 9 - 10, **characterized in that** said agent is to be administered in combination with hormones, preferably selected from the group of:
a) parathyroid hormone-related protein,
b) thyroid hormone,
c) insulin,
d) sex steroids (estrogen, androgen, testosterone),
e) prostaglandins,
f) glucocorticoids.

13. Use according to claim 12, **characterized in that** said agent is to be administered in combination with intermittent administration of parathyroid hormone, preferably in combination with 1,25(OH)₂ vitamin D₃ and analogues or metabolites of vitamin D, calcitonine, estrogen, or bisphosphonates.

14. Use according to one of the claims 2 - 7 or 9 - 13, **characterized in that** said agent is to be administered in combination with vitamins, preferably selected from the group of:
a) 1,25(OH)₂ vitamin D₃ and analogues or metabolites of vitamin D,
b) vitamin C/ascorbate,
c) retinoids.

15. Use according to one of the claims 2 - 7 or 9 - 14, **characterized in that** said agent is to be administered in combination with growth factors, preferably selected from the group of:
a) insulin like growth factors (IGF),
b) transforming growth factor b family (TGF-b),
c) bone morphogenic proteins (BMP),
d) basic fibroblastic growth factor (bFGF),
e) platelet derived growth factor (PDGF),
f) epidermal growth factor (EGF).

16. Use according to one of the claims 2 - 7 or 9 - 15, **characterized in that** said agent is to be administered in combination with cytokines, preferably selected from the group of:
a) interleukins (IL),
b) interferons,
c) leukaemia inhibitory factor (LIF).

17. Use according to one of the claims 2 - 7 or 9 - 16, **characterized in that** said agent is to be administered in combination with matrix proteins, preferably selected from the group of:
A) collagens,
B) glycoproteins,
C) hyaluronan,
D) proteoglycans.

18. Use according to claim 17, **characterized in that** said glycoproteins are selected form the group of:
a) alkaline phosphatase,
b) osteonectin (ON),
c) gamma-carboxy glutamic acid-containing proteins, preferably matrix gla protein or osteocalcin or bone gla protein (OC),
d) arginine-glycine-asparagine-containing proteins, preferably thromspondin, fibronectin, vitronectin, fibrillin, osteoadherin, sialoproteins (osteopontin or bone sialoprotein BSP).

19. Use according to claim 17, **characterized in that** said proteoglycans are selected form the group of:
a) aggrecan,
b) versican,
c) biglycan,
d) decorin.

20. Use according to one of the claims 2 - 7 or 9 - 19, **characterized in that** said agent is to be administered in combination with serum proteins, preferably selected from the group of:
a) albumin
b) alpha-2HS glycoprotein

21. Use according to one of the claims 2 - 7 or 9 - 20, **characterized in that** said agent is to be administered in combination with enzymes, preferably selected from the group of:
a) metalloproteinases,
b) collagenases,
c) gelatinases,
d) stromelysins,
e) plasminogen activators,
f) cysteine proteinases,
g) aspartic proteinases.

22. Use according to one of the claims 2 - 7 or 9 - 20, **characterized in that** said agent is to be administered in combination with calcium salts, bone meal or hydroxyapatite.

23. Use according to one of the claims 2 - 7 or 9 - 21, **characterized in that** said agent can be administered in combination with fluoride salts, preferably sodium fluoride or monosodium fluorophosphate.

24. Use according to one of the claims 2 - 7 or 9 - 23, **characterized in that** said agent is to be administered in combination with peptides, preferably selected from the group of:
a) amylin,
b) vasoactive agents,
c) neuropeptides.

25. Use according to one of the claims 2 - 7 or 9 - 24, **characterized in that** said agent is to be administered in combination with antioxidants, preferably selected from the group of:
a) cysteine,
b) N-acetyl-cysteine,
c) glutathions,
d) vitamins A, C, D or E.

26. Use according to one of the claims 2 - 7 or 9 - 25, **characterized in that** said agent is to be administered in combination with a substance selected from:
a) transferrin,
b) selenium,
c) boron,
d) silicon,
e) nitric oxide.

27. Use according to one of the claims 1 or 3 - 8 or 11 - 26, **characterized in that** said bone cells are selected from the group of osteoblasts, periosteal cells, stromal bone marrow cells, satellite cells of the muscle tissue and mesenchymal stem cells.

28. Use according to one of the claims 1 or 3 - 8 or 11 - 26, **characterized in that** said cartilage cells are selected from the group of chondroblasts, chondroclasts and mesenchymal stem cells.

29. Use according to one of the claims 7 - 8 or 11 - 28, **characterized in that** said bone or cartilage cells are cultured as monolayers, micromass culture or in a three-dimensional, biodegradable scaffold.

30. Use according to claim 29, **characterized in that** said culture of said bone or cartilage cells in said three-dimensional biodegradable scaffold generates a three-dimensional cell assembly having the structure of a seeded sponge, foam or membrane.

31. Use according to claim 29 or 30, **characterized in that** the concentration of said creatine compound in the culture medium is in the range of 10 - 20 mM.

32. Use according to one of the claims 29 to 31, **characterized in that** the culture medium contains 0,1 % to 5,0 %, preferably 0,5 % to 2 % foetal calf serum.

33. Use according to one of the claims 29 to 32 , **characterized in that** the culture medium contains 10 to 250 µg, preferably 25 to 100 µg ascorbic acid or an equivalent amount of a pharmaceutically acceptable ascorbate.

34. Use according to one of the claims 29 to 33 , **characterized in that** said cell culture is started with 2'000 to 100'000 cells, preferably 10'000 to 50'000 cells.

35. Use according to one of the claims 1 to 34 , **characterized in that** said creatine compound or said agent is essentially free of dihydrotriazine.

36. Use according to one of the claims 1 to 35 , **characterized in that** said creatine compound or said agent is essentially free of dicyano-diamide.

37. Use according to one of the claims 1 to 36 , **characterized in that** said creative compound or said agent is essentially free of creatinine as a natural degradation product of creatine.

38. Use according to one of the claims 2 - 7, 9 - 26 or 35 - 37, **characterized in that** said agent is to be administered to a human patient in an amount of 1,4 to 285 mg/kg per day.

39. Use according to one of the claims 2 - 6, 9 - 26 or 35 - 37, **characterized in that** said agent is to be administered to a human patient in an amount of 0,1 to 20,0 g per day.

40. Use according to one of the claims 1 to 39, **characterized in that** said creatine analogue or that agent has the general formula:
Z₁===C(===Z₂)===X-A-Y
and pharmaceutically acceptable salts thereof, wherein:
(a) Y is selected from the group consisting of: -CO₂H, -NHOH, -NO₂, -SO₃H, -C(=O)NHSO₂J and -P(=O)(OH)(OJ), wherein J is selected from the group consisting of: hydrogen, C₁-C₆ straight chain alkyl, C₃-C₆ branched alkyl, C₂-C₆ straight alkenyl, C₃-C₆ branched alkenyl and aryl;
(b) A is selected from the group consisting of: C, CH, C₁-C₅ alkyl, C₂-C₅ alkenyl, C₂-C₅ alkynyl, and C₁-C₅ alkoyl chain, each having 0-2 substituents which are selected independently from the group consisting of:
(1) K, where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, C₄-C₆ branched alkoyl, K having 0-2 substituents independently selected from the group consisting of: bromo, chloro, epoxy and acetoxy;
(2) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy; and
(3) -NH-M, wherein M is selected from the group consisting of: hydrogen, C₁-C₄ alkyl, C₂-C₄ alkenyl, C₁-C₄ alkoyl, C₃-C₄ branched alkyl, C₃-C₄ branched alkenyl, and C₄-C₆ branched alkoyl;
(c) X is selected from the group consisting of: NR₁, CHR₁, CR₁, O and S, wherein R₁ is selected from the group consisting of
(1) hydrogen;
(2) K where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
(3) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
(4) a C₅-C₉ Alpha-amino-omega-methyl-omega-adenosylcarboxylic acid attached via the omega-methyl carbon;
(5) a C₅-C₉ Alpha-amino-omega-aza-omega-methyl-omega -adenosylcarboxylic acid attached via the omega-methyl carbon; and
(6) a C₅-C₉ Alpha-amino-omega-thia-omega-methyl-omega-adenosylcarboxylic acid wherein A and X are connected by a single or double bond;
(d) Z₁ and Z₂ are chosen independently from the group consisting of: =O, -NHR₂, -CH₂R₂, -NR₂OH; wherein, Z₁ and Z₂ may not both be =O and wherein R₂ is selected from the group consisting of:
(1) hydrogen;
(2) K, where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of bromo, chloro, epoxy and acetoxy;
(3) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
(4) a C₄-C₈ Alpha-amino-carboxylic acid attached via the omega-carbon;
(5) B, wherein B is selected from the group consisting of: -CO₂H, -NHOH, -NO₂, -SO₃H, -C(=O)NHSO₂J and -P(=O) (OH) (OJ), wherein J is selected from the group consisting of: hydrogen C₁-C₆ straight alkyl, C₃-C₆ branched alkyl, C₂-C₆ straight alkenyl, C₃-C₆ branched alkenyl and aryl; wherein B is optionally connected to the nitrogen via a linker selected from the group consisting of: C₁-C₂ alkyl, C₂ alkenyl, and C₁-C₂ alkoyl;
(6) -D-E, wherein D is selected from the group consisting of: C₁-C₃ straight chain alkyl, C₃ branched alkyl, C₂-C₃ straight alkenyl, C₃ branched alkenyl, C₁-C₃ straight alkoyl, and aryl; and E is selected from the group consisting of: -(PO₃)ₙNMP, where n is 0-2 and NMP is a ribonucleotide monophosphate connected via the 5'-phosphate, 3'-phosphate or the aromatic ring of the base; -[P(=O)(OCH₃)(O)]ₘ-Q, wherein m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose of the aromatic ring of the base; and an aryl group containing 0-3 substituents chosen independently from the group consisting of: Cl, Br, epoxy, acetoxy, -OG, -C(=O)G, and -CO₂G, where G is independently selected from the group consisting of: C₁-C₆ straight alkyl, C₂-C₆ straight alkenyl, C₁-C₆ straight alkoyl, C₃-C₆ branched alkyl, C₁-C₆ branched alkenyl, C₄-C₆ branched alkoyl; wherein E may be attached at any point to D, and if D is alkyl or alkenyl, D may be connected at either or both ends by an amide linkage; and
(7) -E, wherein E is selected from the group consisting of: -(PO₃)ₙNMP, where n is 0-2 and NMP is a ribonucleotide monophosphate connected via the 5'-phosphate, 3'-phosphate or the aromatic ring of the base; -P(P(=O)(OCH₃)(O))ₘ-Q, wherein m is 0-3 and Q is a ribonucleoside connected via the ribose or the aromatic ring of the base; -[P(=O)(OH)(CH₂)]ₘ-Q, wherein m is 0-3 and Q is a ribonucleoside connected via the ribose of the aromatic ring of the base; and an aryl group containing 0-3 substituents chosen independently from the group consisting of: Cl, Br, epoxy, acetoxy, -OG, -C(=O)G, and -CO₂G, where G is independently selected from the group consisting of: C₁-C₆ straight alkyl, C₂-C₆ straight alkenyl, C₁-C₆ straight alkoyl; C₃-C₆ branched alkyl, C₃-C₆ branched alkenyl, C₄-C₆ branched alkoyl; and if E is aryl, E may be connected by an amide linkage;
(e) if R₁ and at least one R₂ group are present, R₁ may be connected by a single or double bond to an R₂ group to form a cycle of 5 to 7 members;
(f) is two R₂ groups are present, they may be connected by a single or double bond to form a cycle of 5 to 7 members; and
(g) if R₁ is present and Z₁ or Z₂ is selected from the group consisting of -NHR₂, -CH₂R₂ and -NR₂OH, then R₁ may be connected by a single or double bond to the carbon or nitrogen of either Z₁ or Z₂ to form a cycle of 4 to 7 members.

41. Use according to one of the claims 2 - 7 or 9 - 40, **characterized in that** said creatine compound or said agent is used for the treatment of degenerative diseases of cartilage, in particular of arthritis.

42. Use according to one of the claims 1 to 41, **characterized in that** said creatine compounds or said agent are combined with creatine kinase transfection of bone or cartilage cells.

43. Use according to one of the claims 2 - 6, 9 - 26 or 35 - 42, **characterized in that** said agent is admixed with pharmacologically suitable carrier substances to improve bioavailability.

44. Use according to claim 43, **characterized in that** said carrier substance is selected from the group of carbohydrates, in particular from maltodextrins and/or dextrose.

45. Use of creatine in combination with genetic engineering of cartilage or bone cells transfected to overexpress the enzyme creatine kinase, for the preparation of an agent for the treatment of bone or cartilage cells and tissues, **characterized in that**
a) bone or cartilage forming cells removed from a healthy individual or a patient are brought into cell culture in the presence of creatine and transfected with complementary DNA coding for creatine kinase isoforms and made to overexpress creatine kinase isoenzyme(s);
b) the metabolically engineered cells obtained in step a) are then expanded and cultivated to form in vitro genetically engineered cartilage or bone tissues transplantable into areas of cartilage or bone defects of said healthy individual or said patient.

## Patentansprüche

1. Verwendung von Kreatinverbindungen, einschliesslich Analoga oder pharmazeutisch akzeptable Salze derselben, zur *In-vitro*-Behandlung von Knochenzellen, Knorpelzellen oder mesenchymalen Stammzellen und Geweben, welche für die Transplantation in den tierischen oder menschlichen Körper bestimmt sind.

2. Verwendung von Kreatinverbindungen, einschliesslich Analoga oder pharmazeutisch akzeptable Salze derselben, zur Zubereitung eines Wirkstoffes für die In-vitro-Behandlung von Knochen- und Knorpelerkrankungen.

3. Verwendung nach Anspruch 2, **dadurch gekennzeichnet, dass** die Kreatinverbindung bzw. der Wirkstoff zur therapeutischen Behandlung von Knochen- und Knorpelerkrankungen, vorzugsweise von Osteoporose, Osteoarthritis oder Periodontitis, bzw. zur prophylaktischen Behandlung dieser Knochen- bzw. Knorpelerkrankungen verwendet wird.

4. Verwendung nach Anspruch 1 oder 2, **dadurch gekennzeichnet, dass** die Kreatinverbindung bzw. der Wirkstoff zur Förderung des Wachstums und der Mineralisierung von Knochen- bzw. Knorpelzellen und - geweben verwendet wird.

5. Verwendung nach Anspruch 1 oder 2, **dadurch gekennzeichnet, dass** die Kreatinverbindung bzw. der Wirkstoff zur konservativen oder operativen Behandlung von Knochenfrakturen oder Knochendefekten verwendet wird.

6. Verwendung nach Anspruch 5, **dadurch gekennzeichnet, dass** die Kreatinverbindung bzw. der Wirkstoff in ein auf die Fraktur oder auf den Defekt aufzubringendes Knochen- oder Knorpeltransplantat integriert ist.

7. Verwendung nach Anspruch 5, **dadurch gekennzeichnet, dass** die Kreatinverbindung bzw. der Wirkstoff in die dreidimensionalen Strukturelemente von Osteoblasten, Chondrozyten oder mesenchymalen Stammzelle eingebaut werden, welche zur gewebetechnologischen Aufbereitung der Knorpel- und Knochendefekte bestimmt sind.

8. Verwendung nach Anspruch 1, **dadurch gekennzeichnet, dass** knochen- bzw. knorpelbildende Zellen, welche aus einem gesunden Individuum oder aus dem Körper eines bestimmten Patienten gewonnenen worden sind, unter Beigabe des Wirkstoffes zu einem dreidimensionalen Zellagglomerat kultiviert werden, das in einem späteren Verfahrensschritt an eine bestimmte von einem Knochen- bzw. Knorpeldefekt betroffene Körperstelle desselben Patienten übertragbar ist.

9. Verwendung nach Anspruch 2, **dadurch gekennzeichnet, dass** der Wirkstoff dazu verwendet wird, um die Annahme und das Einwachsen des Knochentransplantats zu verbessern.

10. Verwendung nach Anspruch 9, **dadurch gekennzeichnet, dass** es sich bei den Knochenimplantaten um Endoprothesen, vorzugsweise um Gelenk-Endoprothesen handelt.

11. Verwendung nach einem der Ansprüche 1 bis 10, **dadurch gekennzeichnet, dass** die Kreatinverbindung bzw. der Wirkstoff aus der Gruppe bestehend aus Kreatin, Kreatinphosphat, Kreatinpyruvat, Cyclokreatin, Homokreatin oder Homocyclokreatin ausgewählt ist.

12. Verwendung nach einem der Ansprüche 2 - 7 oder 9 - 10, **dadurch gekennzeichnet, dass** der Wirkstoff in Kombination mit Hormonen zu verabreichen ist, die vorzugsweise aus der Gruppe der folgenden Elemente ausgewählt sind:
a) PTH-verwandtes Protein,
b) Schilddrüsenhormon,
c) Insulin,
d) steroidische Geschlechtshormone (Östrogen, Androgen, Testosteron),
e) Prostaglandine,
f) Glucocorticoide.

13. Verwendung nach Anspruch 12, **dadurch gekennzeichnet, dass** der Wirkstoff in Kombination mit einer intermittierenden Verabreichung von Nebenschilddrüsenhormon, vorzugsweise in Kombination mit 1,25(OH)₂ Vitamin D₃ und Analoga bzw. Metaboliten von Vitamin D, Calcitonin, Östrogen oder Biophosphonaten zu verabreichen ist.

14. Verwendung nach einem der Ansprüche 2 - 7 oder 9 -13, **dadurch gekennzeichnet, dass** der Wirkstoff in Kombination mit Vitaminen zu verabreichen ist, die vorzugsweise aus der Gruppe der folgenden Elemente ausgewählt sind:
a) 1,25(OH)₂ Vitamin D₃ und Analoga bzw. Metaboliten von Vitamin D,
b) Vitamin C/Ascorbat,
c) Retinoiden.

15. Verwendung nach einem der Ansprüche 2 - 7 oder 9 -14, **dadurch gekennzeichnet, dass** der Wirkstoff in Kombination mit Wachstumsfaktoren zu verabreichen ist, die vorzugsweise aus der Gruppe der folgenden Elemente ausgewählt sind:
a) insulinartiger Wachstumsfaktor (IGF),
b) b-Wachstumsfaktor (TGF-b),
c) knochenformbildende Proteine (BMP),
d) basischer Fibroblasten-Wachstumsfaktor (bFGF),
e) blutplättchenabgeleiteter Wachstumsfaktor (PDFG);
f) epidermaler Wachstumsfaktor (EGF).

16. Verwendung nach einem der Ansprüche 2 - 7 oder 9 -15, **dadurch gekennzeichnet, dass** der Wirkstoff in Kombination mit Zytokinen zu verabreichen ist, die vorzugsweise aus der Gruppe der folgenden Elemente ausgewählt sind:
a) Interleukine (IL),
b) Interferone,
c) leukämiehemmender Faktor (LIF).

17. Verwendung nach einem der Ansprüche 2 - 7 oder 9 -16, **dadurch gekennzeichnet, dass** der Wirkstoff in Kombination mit Matrixproteinen zu verabreichen ist, die vorzugsweise aus der Gruppe der folgenden Elemente ausgewählt sind:
A) Kollagene,
B) Glycoproteine,
C) Hyaluronan,
D) Proteoglykane.

18. Verwendung nach Anspruch 17, **dadurch gekennzeichnet, dass** die Glycoproteine aus der Gruppe der folgenden Elemente ausgewählt sind:
a) alkalische Phosphatase
b) Osteonectin (ON),
c) Gamma-Carboxy-Glutaminsäure enthaltende Proteine, vorzugsweise Matrix gla Protein oder Osteokalzin oder Knochen gla Protein (OC),
d) Arginin-Glycin-Asparagin enthaltende Proteine, vorzugsweise Thromspondin, Fibronectin, Vitronectin, Fibrillin, Osteoadherin, Sialoproteine (Osteopontin oder Knochen-Sialoprotein BSP).

19. Verwendung nach Anspruch 17, **dadurch gekennzeichnet, dass** die Proteoglykane aus der Gruppe der folgenden Elemente ausgewählt sind:
a) Aggrecan,
b) Versican,
c) Biglycan,
d) Decorin.

20. Verwendung nach einem der Ansprüche 2 - 7 oder 9 -19, **dadurch gekennzeichnet, dass** der Wirkstoff in Kombination mit Serumproteinen zu verabreichen ist, die vorzugsweise aus der Gruppe der folgenden Elemente ausgewählt sind:
a) albumin
b) Alpha-2HS Glykoprotein.

21. Verwendung nach einem der Ansprüche 2 - 7 oder 9 -20, **dadurch gekennzeichnet, dass** der Wirkstoff in Kombination mit Enzymen zu verabreichen ist, die vorzugsweise aus der Gruppe der folgenden Elemente ausgewählt sind:
a) Metalloproteasen,
b) Kollagenasen,
c) Gelatinasen,
d) Stromelysine,
e) Plasminogenaktivatoren,
f) Cysteinproteinasen,
g) Asparaginproteinasen.

22. Verwendung nach einem der Ansprüche 2 - 7 oder 9 -20, **dadurch gekennzeichnet, dass** der Wirkstoff in Kombination mit Calciumsalzen, Knochenmehl oder Hydroxyapatit zu verabreichen ist.

23. Verwendung nach einem der Ansprüche 2 - 7 oder 9 -21, **dadurch gekennzeichnet, dass** der Wirkstoff in Kombination mit Fluoridsalzen, vorzugsweise mit Natriumfluorid oder Mononatriumfluorophosphat zu verabreichen ist.

24. Verwendung nach einem der Ansprüche 2 - 7 oder 9 -23, **dadurch gekennzeichnet, dass** der Wirkstoff in Kombination mit Peptiden zu verabreichen ist, die vorzugsweise aus der Gruppe der folgenden Elemente ausgewählt sind:
a) Amylin,
b) vasoaktive Wirkstoffe,
c) Neuropeptide.

25. Verwendung nach einem der Ansprüche 2 - 7 oder 9 -24, **dadurch gekennzeichnet, dass** der Wirkstoff in Kombination mit Antioxidantia zu verabreichen ist, die vorzugsweise aus der Gruppe der folgenden Elemente ausgewählt sind:
a) Cystein,
b) N-Acetyl-Cystein,
c) Glutathione,
d) die Vitamine A, C, D oder E.

26. Verwendung nach einem der Ansprüche 2 - 7 oder 9 -25, **dadurch gekennzeichnet, dass** der Wirkstoff in Kombination mit einer Substanz zu verabreichen ist, die aus den folgenden Elementen ausgewählt ist:
a) Transferrin,
b) Selen,
c) Bor,
d) Silikon,
e) Stickstoffoxyd.

27. Verwendung nach einem der Ansprüche 1 oder 3 - 8 oder 11 - 26, **dadurch gekennzeichnet, dass** die Knochenzellen aus der Gruppe der Osteoblaste, Periostzellen, Knochenmark-Stromazellen, Mantelzellen des Muskelgewebes und mesenchymalen Stammzellen ausgewählt sind.

28. Verwendung nach einem der Ansprüche 1 oder 3 - 8 oder 11 - 26, **dadurch gekennzeichnet, dass** die Knorpelzellen aus der Gruppe der Chondroblaste, Chondroklaste und mesenchymalen Stammzellen ausgewählt sind.

29. Verwendung nach einem der Ansprüche 7 - 8 oder 11 - 28, **dadurch gekennzeichnet, dass** die Knochen- oder Knorpelzellen als Monoschicht-Kulturen, Mikromasse-Kulturen oder in einem dreidimensionalen, biologisch abbaubaren Stützgewebe herangezüchtet werden.

30. Verwendung nach Anspruch 29, **dadurch gekennzeichnet, dass** die Kultur der Knochen- bzw. Knorpelzellen in dem dreidimensionalen, biologisch abbaubaren Stützgewebe ein dreidimensionales Zellagglomerat bilden welches die Struktur eines beimpften Schwamms, eines Schaums oder einer Membran aufweisen.

31. Verwendung nach Anspruch 29 oder 30, **dadurch gekennzeichnet, dass** die Konzentration der Kreatinverbindung in dem Kulturmedium in einem Bereich zwischen 10 - 20 mM liegt.

32. Verwendung nach einem der Ansprüche 29 bis 31, **dadurch gekennzeichnet, dass** das Kulturmedium 0,1 % bis 5,0 %, vorzugsweise 0,5 % bis 2 % fötales Kälberserum enthält.

33. Verwendung nach einem der Ansprüche 29 bis 32, **dadurch gekennzeichnet, dass** das Kulturmedium 10 bis 250 µg, vorzugsweise 25 bis 100 µg Ascorbinsäure bzw. eine entsprechende Menge eines pharmazeutisch akzeptablen Ascorbats enthält.

34. Verwendung nach einem der Ansprüche 29 bis 33, **dadurch gekennzeichnet, dass** die Zellkultur mit einer Menge von 2 000 bis 100 000 Zellen, vorzugsweise 10 000 bis 50 000 Zellen begonnen wird.

35. Verwendung gemäss einem der Ansprüche 1 bis 34, **dadurch gekennzeichnet, dass** die Kreatinverbindung bzw. der Wirkstoff im wesentlichen frei von Dihydrotriazin ist.

36. Verwendung gemäss einem der Ansprüche 1 bis 35, **dadurch gekennzeichnet, dass** die Kreatinverbindung bzw. der Wirkstoff im wesentlichen frei von Dicyanodiamidist.

37. Verwendung gemäss einem der Ansprüche 1 bis 36, **dadurch gekennzeichnet, dass** die Kreatinverbindung bzw. der Wirkstoff im wesentlichen frei von Kreatinin, einem natürlichen Zersetzungsprodukt von Kreatin, ist.

38. Verwendung nach einem der Ansprüche 2 - 7, 9 - 26 oder 35 - 37, **dadurch gekennzeichnet, dass** der Wirkstoff einem menschlichen Patienten in einer Menge von 1,4 bis 285 mg/kg pro Tag zu verabreichen ist.

39. Verwendung nach einem der Ansprüche 2 -6, 9 - 26 oder 35 - 37, **dadurch gekennzeichnet, dass** der Wirkstoff einem menschlichen Patienten in einer Menge von 0,1 bis 20,0 g pro Tag zu verabreichen ist.

40. Verwendung gemäss einem der Ansprüche 1 bis 39, **dadurch gekennzeichnet, dass** das Kreatinanalogon bzw. der Wirkstoff die folgende allgemeine Formel aufweist:
Z₁===C(===Z₂)===X-A-Y
und auch dessen pharmazeutisch akzeptable Salze beinhaltet, wobei:
(a) Y aus der Gruppe bestehend aus: -CO₂H, -NHOH, -NO₂, -SO₃H, -C(=O)NHSO₂J und -P(=O) (OH) (OJ) ausgewählt ist, wobei J aus der Gruppe bestehend aus: Wasserstoff, C₁-C₆ geradkettigem Alkyl, C₃-C₆ verzweigtem Alkyl, C₂-C₆ linearem Alkenyl, C₃-C₆ verzweigtem Alkenyl und Aryl ausgewählt ist;
(b) A aus der Gruppe bestehend aus: C, CH, C₁-C₅ Alkyl, C₂-C₅ Alkenyl, C₂-C₅ Alkinyl, und der C₁-C₅ Alkoylkette ausgewählt ist, wobei ein jedes dieser Elemente 0-2 Substituenten hat, die unabhängig voneinander aus der Gruppe bestehend aus den folgenden Elementen ausgewählt ist:
(1) K, wobei K aus der Gruppe bestehend aus: C₁-C₆ linearem Alkyl, C₂-C₆ linearem Alkenyl, C₁-C₆ linearem Alkoyl, C₃-C₆ verzweigtem Alkyl, C₃-C₆ verzweigtem Alkenyl, C₄-C₆ verzweigtem Alkoyl ausgewählt ist, wobei K 0-2 Substituenten hat, die unabhängig voneinander aus der Gruppe bestehend aus: Bromo-, Chloro-, Epoxy- und Acetoxy- ausgewählt sind;
(2) einer Arylgruppe, welche aus der Gruppe bestehend aus: einem 1-2 Ring Carbocyclus und einem 1-2 Ring Heterocyclus ausgewählt ist, wobei die Arylgruppe 0-2 Substituenten enthält, die unabhängig voneinander aus der Gruppe bestehend aus: -CH₂L und -COCH₂L ausgewählt sind, wobei L unabhängig aus der Gruppe bestehend aus: Bromo-, Chloro-, Epoxy- und Acetoxy- ausgewählt ist; und
(3) -NH-M, wobei M aus der Gruppe bestehend aus: Wasserstoff, C₁-C₄ Alkyl, C₂-C₄ Alkenyl, C₁-C₄ Alkoyl, C₃-C₄ verzweigtem Alkyl, C₃-C₄ verzweigtem Alkenyl, und C₄-C₆ verzweigtem Alkoyl ausgewählt ist;
(c) X aus der Gruppe bestehend aus: NR₁, CHR₁, CR₁, O und S ausgewählt ist, wobei R₁ aus der Gruppe bestehend aus den folgenden Elementen ausgewählt ist
(1) Wasserstoff;
(2) K, wobei K aus der Gruppe bestehend aus: C₁-C5 linearem Alkyl, C₂-C₆ linearem Alkenyl, C₁-C₆ linearem Alkoyl, C₃-C₆ verzweigtem Alkyl, C₃-C₆ verzweigtem Alkenyl, und C₄-C₆ verzweigtem Alkoyl ausgewählt ist, wobei K 0-2 Substituenten hat, die unabhängig voneinander aus der Gruppe bestehend aus: Bromo-, Chloro-, Epoxy- und Acetoxy- ausgewählt sind;
(3) einer Arylgruppe, welche aus der Gruppe bestehend aus: einem 1-2 Ring Carbocyclus und einem 1-2 Ring Heterocyclus ausgewählt ist, wobei die Arylgruppe 0-2 Substituenten enthält, die unabhängig voneinander aus der Gruppe bestehend aus: -CH₂L und -COCH₂L ausgewählt sind, wobei L unabhängig aus der Gruppe bestehend aus: Bromo-, Chloro-, Epoxy- und Acetoxy- ausgewählt ist;
(4) einer C₅-C₉ Alpha-amino-omega-methyl-omega-Adenosyl-Carbonsäure, welche am Omega-methyl-Carbon hängt;
(5) einer C₅-C₉ Alpha-amino-omega-aza-omega-methylomega-Adenosyl-Carbonsäure, welche am Omega-methyl-Carbon hängt;
(6) einer C₅-C₉ Alpha-amino-omega-thia-omega-methylomega-Adenosyl-Carbonsäure, wobei A und X mittels einer Einfach- bzw. einer Doppelbindung verbunden sind;
(d) Z₁ und Z₂ unabhängig voneinander aus der Gruppe bestehend aus: =O, -NHR₂, -CH₂R₂, -NR₂OH ausgewählt sind; wobei Z₁ und Z₂ nicht beide =O sein dürfen und wobei R₂ aus der Gruppe bestehend aus den folgenden Elementen ausgewählt ist:
(1) Wasserstoff;
(2) K, wobei K aus der Gruppe bestehend aus: C₁-C₆ linearem Alkyl, C₂-C₆ linearem Alkenyl, C₁-C₆ linearem Alkoyl, C₃-C₆ verzweigtem Alkyl, C₃-C₆ verzweigtem Alkenyl, und C₄-C₆ verzweigtem Alkoyl ausgewählt ist, wobei K 0-2 Substituenten hat, die unabhängig voneinander aus der Gruppe bestehend aus Bromo-, Chloro-, Epoxy- und Acetoxy- ausgewählt sind;
(3) einer Arylgruppe, welche aus der Gruppe bestehend aus: einem 1-2 Ring Carbocyclus und einem 1-2 Ring Heterocyclus ausgewählt ist, wobei die Arylgruppe 0-2 Substituenten enthält, die unabhängig voneinander aus der Gruppe bestehend aus: -CH₂L und -COCH₂L ausgewählt sind, wobei L unabhängig aus der Gruppe bestehend aus: Bromo-, Chloro-, Epoxy- und Acetoxy- ausgewählt ist;
(4) einer C₄-C₈ Alpha-amino-Carbonsäure, welche am Omega-Carbon hängt;
(5) B, wobei B aus der Gruppe bestehend aus: -CO₂H, -NHOH, -NO₂, -SO₃H, -C{=O)NHSO₂J und -P(=O) (OH) (OJ) ausgewählt ist, wobei J aus der Gruppe bestehend aus: Wasserstoff, C₁-C₆ linearem Alkyl, C₃-C₆ verzweigtem Alkyl, C₂-C₆ verzweigtem Alkenyl, C₃-C₆ verzweigtem Alkenyl und Aryl ausgewählt ist; wobei B mit dem Stickstoff wahlweise über einen Bindungsvermittler verbunden ist, der aus der Gruppe bestehend aus: C₁-C₂ Alkyl, C₂ Alkenyl, und C₁-C₂ Alkoyl ausgewählt ist;
(6) -D-E, wobei D aus der Gruppe bestehend aus: C₁-C₃ geradkettigem Alkyl, C₃ verzweigtem Alkyl, C₂-C₃ linearem Alkenyl, C₃ verzweigtem Alkenyl, C₁-C₃ linearem Alkoyl, und Aryl ausgewählt ist; und E aus der Gruppe bestehend aus: -(PO₃)ₙNMP ausgewählt ist, wobei n gleich 0-2 ist und NMP ein Ribonukleotid-Monophosphat ist, das an dem 5'-Phosphat, an dem 3'-Phosphat oder an dem aromatischen Ring der Base hängt; -[P(=O) (OCH₃) (O)]ₘ-Q, wobei m gleich 0-3 ist und Q ein Ribonukleosid ist, das an der Ribose oder an dem aromatischen Ring der Base hängt; -[P(==O) (OH) (CH₂) ]ₘ-Q, wobei m gleich 0-3 ist und Q ein Ribonukleosid ist, das an der Ribose oder an dem aromatischen Ring der Base hängt; und einer Arylgruppe, die 0-3 Substituenten hat, welche unabhängig voneinander aus der Gruppe bestehend aus: Cl, Br, Epoxy-, Acetoxy-, -OG, -C(=O)G, und -CO₂G ausgewählt sind, wobei G unabhängig aus der Gruppe bestehend aus: C₁-C6 linearem Alkyl, C₂-C₆ linearem Alkenyl, C₁-C₆ linearem Alkoyl, C₃-C₆ verzweigtem Alkyl, C₁-C₆ verzweigtem Alkenyl, C₄ - C₆ verzweigtem Alkoyl ausgewählt ist; wobei E an jedem beliebigen Punkt mit D verbunden sein kann, und - sofern D Alkyl oder Alkenyl ist - D an einem beliebigen oder an beiden Enden durch eine Amidbindung verbunden sein kann; und
(7) -E, wobei E aus der Gruppe bestehend aus: -(PO₃)ₙNMP ausgewählt ist, wobei n gleich 0-2 ist und MMP ein Ribonukleotid-Monophosphat ist, das an dem 5'-Phosphat, an dem 3'-Phosphat oder an dem aromatischen Ring der Base hängt; -P(P(=O)(OCH₃)(O))ₘ-Q, wobei m gleich 0-3 ist und Q ein Ribonukleosid ist, das an der Ribose oder an dem aromatischen Ring der Base hängt; -[P(=O)(OH) (CH₂)]ₘ-Q, wobei m gleich 0-3 ist und Q ein Ribonukleosid ist, das an der Ribose oder an dem aromatischen Ring der Base hängt; und einer Arylgruppe, welche 0-3 Substituenten hat, welche unabhängig voneinander aus der Gruppe bestehend aus: Cl, Br, Epoxy-, Acetoxy-, -OG, -C(=O)G, und -CO₂G ausgewählt sind, wobei G unabhängig aus der Gruppe bestehend aus: C₁-C₆ linearem Alkyl, C₂-C₆ linearem Alkenyl, C₁-C₆ linearem Alkoyl; C₃-C₆ verzweigtem Alkyl, C₃-C₆ verzweigtem Alkenyl, C₄-C₆ verzweigtem Alkoyl ausgewählt ist; und - sofern E Aryl ist - E durch eine Amidbindung verbunden sein kann;
(e) sofern R₁ und zumindest eine R₂-Gruppe vorhanden sind, R₁ durch einen Einfach- oder eine Doppelbindung mit einer R₂-Gruppe zu einem Zyklus aus 5 bis 7 Bestandteilen verbunden sein kann;
(f) sofern zwei R₂-Gruppen vorhanden sind, diese durch eine Einfach- oder eine Doppelbindung miteinander zu einem Zyklus aus 5 bis 7 Bestandteilen verbunden sein können; und
(g) sofern R₁ vorhanden ist und Z₁ oder Z₂ aus der Gruppe bestehend aus -NHR₂, -CH₂R₂ und -NR₂OH ausgewählt ist, R₁ durch eine Einfach- oder eine Doppelbindung mit dem Kohlenstoff oder dem Stickstoff von Z₁ bzw. Z₂ zu einem Zyklus aus 4 bis 7 Bestandteilen verbunden sein kann.

41. Verwendung nach einem der Ansprüche 2 - 7 oder 9 - 40, **dadurch gekennzeichnet, dass** die Kreatinverbindung bzw. der Wirkstoff zur Behandlung degenerativer Knorpelerkrankungen, insbesondere zur Behandlung von Arthritis verwendet wird.

42. Verwendung nach einem der Ansprüche 1 bi 41, **dadurch gekennzeichnet, dass** die Kreatinverbindung bzw. der Wirkstoff mit der Kreatinkinase-Transfektion von Knochenoder Knorpelzellen kombiniert wird.

43. Verwendung nach einem der Ansprüche 2 - 6, 9 - 26 oder 35 - 42, **dadurch gekennzeichnet, dass** der Wirkstoff mit pharmakologisch geeigneten Trägersubstanzen vermischt wird, um eine bessere Bioverfügbarkeit zu gewährleisten.

44. Verwendung nach Anspruch 43, **dadurch gekennzeichnet, dass** die Trägersubstanz aus der Gruppe der Kohlenhydrate, insbesondere aus den Maltodextrinen und/oder Dextrose ausgewählt ist.

45. Verwendung von Kreatin in Kombination mit der gentechnologischen Aufbereitung von Knorpel- oder Knochenzellen, die entsprechend transfiziert und damit zu einer Überexpression des Enzyms Kreatinkinase veranlasst werden, und zwar im Hinblick auf die Zubereitung eines Wirkstoffes zur Behandlung von Knochen- und Knorpelzellen und -geweben, **dadurch gekennzeichnet, dass**
a) ausgehend von knochen- oder knorpelbildende Zellen, die von einem gesunden Individuum bzw. aus dem Körper des Patienten entnommen worden sind, unter Beigabe von Kreatin eine Zellkultur angelegt wird und dass die Zellen mit komplementärer DNA-Codierung für Creatinkinase-Isoformen transfiziert werden und zu einer Überexpression eines oder mehrerer Kreatinkinase-Isoenzyme veranlasst werden;
b) die unter Schritt a) gewonnenen, stoffwechselveränderten Zellen in der Folge expandiert und kultiviert werden, um *in vitro* gentechnologisch aufbereitete Knorpel- oder Knochengewebe zu bilden, welche in die von Knorpel- bzw. Knochendefekten betroffenen Bereiche des jeweiligen gesunden Individuums bzw. des Patienten transplantierbar sind.

## Revendications

1. Utilisation de composés de créatine, y compris des analogues ou des sels pharmaceutiquement acceptables de celle-ci, pour le traitement in vitro de cellules osseuses, de cellules cartilagineuses ou de cellules souches mésenchymales et de tissus pour transplantation sur des animaux ou des humains.

2. Utilisation de composés de créatine, y compris des analogues ou des sels pharmaceutiquement acceptables de celle-ci, pour la préparation d'un agent pour le traitement in vivo de maladies des os ou des cartilages.

3. Utilisation selon la revendication 2, **caractérisée en ce que** ledit composé de créatine ou ledit agent sont utilisés pour le traitement thérapeutique de maladies des os ou des cartilages, de préférence de l'ostéoporose, de l'ostéoarthrite ou de la périodontite, ou pour le traitement prophylactique desdites maladies des os ou des cartilages.

4. Utilisation selon la revendication 1 ou 2, **caractérisée en ce que** ledit composé de créatine ou ledit agent sont utilisés pour stimuler la croissance et la minéralisation de cellules et de tissus osseux ou cartilagineux.

5. Utilisation selon la revendication 1 ou 2, **caractérisée en ce que** ledit composé de créatine ou ledit agent sont utilisés pour les traitements conservateurs ou opératoires de fractures osseuses ou de lésions osseuses.

6. Utilisation selon la revendication 5, **caractérisée en ce que** ledit composé de créatine ou ledit agent sont incorporés dans une greffe osseuse ou cartilagineuse à appliquer sur lesdites fractures ou lésions.

7. Utilisation selon la revendication 5, **caractérisée en ce que** ledit composé de créatine ou ledit agent sont incorporés à des constructions tridimensionnelles d'ostéoblastes, de chondrocytes ou de cellules souches mésenchymales destinées à l'ingénierie tissulaire desdites lésions osseuses ou cartilagineuses.

8. Utilisation selon la revendication 1, **caractérisée en ce que** des cellules de formation d'os ou de cartilages prélevées sur un individu sain ou sur un patient particulier sont cultivées en présence dudit agent pour former un assemblage tridimensionnel de cellules qui peut, dans une étape subséquente, être transféré en un site spécifique de lésion osseuse ou cartilagineuse du même patient particulier.

9. Utilisation selon la revendication 2, **caractérisée en ce que** ledit agent est utilisé pour améliorer la tolérance et l'intégration osseuse d'implants osseux.

10. Utilisation selon la revendication 9, **caractérisée en ce que** lesdits implants osseux sont des endoprothèses, de préférence des endoprothèses articulaires.

11. Utilisation selon l'une des revendications 1 à 10, **caractérisée en ce que** ledit composé de créatine ou ledit agent sont choisis dans le groupe formé par la créatine, la créatine phosphate, la créatine pyruvate, la cyclocréatine, l'homocréatine ou l'homocyclocréatine.

12. Utilisation selon l'une des revendication 2 - 7 ou 9 - 10, **caractérisée en ce que** ledit agent est à administrer en combinaison avec des hormones, de préférence choisies dans le groupe formé par:
a) des protéines apparentées à l'hormone parathyroïde,
b) des hormones thyroïdiennes,
c) l'insuline,
d) des stéroïdes sexuels (oestrogènes, androgènes, testostérone),
e) des prostaglandines,
f) des glucocorticoïdes.

13. Utilisation selon la revendication 12, **caractérisée en ce que** ledit agent est à administrer en combinaison avec une administration intermittente d'hormone parathyroïde, de préférence en combinaison avec de la vitamine 1,25 (OH)₂ D3 et des analogues ou des métabolites de la vitamine D, de la calcitonine, des oestrogènes ou des bisphosphonates.

14. Utilisation selon l'une des revendications 2 - 7 ou 9 - 13, **caractérisée en ce que** ledit agent est à administrer en combinaison avec des vitamines, de préférence choisies dans le groupe formé par:
a) la vitamine 1,25 (OH)₂ D3 et des analogues ou des métabolites de la vitamine D,
b) de la vitamine C / ascorbate,
c) des rétinoïdes.

15. Utilisation selon l'une des revendications 2 - 7 ou 9 - 14, **caractérisée en ce que** ledit agent est à administrer en combinaison avec des facteurs de croissance, de préférence choisis dans le groupe formé par:
a) des facteurs de croissance analogues à l'insuline (IGF),
b) la famille du facteur de croissance transformant b (TGF-b),
c) des protéines morphogéniques osseuses (BMP),
d) du facteur de croissance fibroblastique basique (bFGF),
e) du facteur de croissance dérivé de plaquettes (PDGF),
f) du facteur de croissance épidermique (EGF).

16. Utilisation selon l'une des revendications 2 - 7 ou 9 - 15, **caractérisée en ce que** ledit agent est à administrer en combinaison avec des cytokines, de préférence choisies dans le groupe formé par:
a) des interleucines (IL),
b) des interférons,
c) du facteur inhibiteur de la leucémie (LIF).

17. Utilisation selon l'une des revendications 2 - 7 ou 9 - 16, **caractérisée en ce que** ledit agent est à administrer en combinaison avec des protéines matricielles, de préférence choisies dans le groupe formé par:
a) des collagènes,
b) des glycoprotéines,
c) de l'hyaluronane,
d) des protéoglycans.

18. Utilisation selon la revendication 17, **caractérisée en ce que** lesdites glycoprotéines sont choisies dans le groupe formé par:
a) des phosphatases alcalines,
b) de l'ostéonectine (ON).
c) des protéines contenant de l'acide γ-carboxyglutamique, de préférence la gla-protéine matricielle ou de l'ostéocalcine ou gla-protéine osseuse (OC),
d) des protéines contenant de l'arginine - glycine - asparagine, de préférence la thrombospondine, la fibronectine, la vitronectine, la fibrilline, l'ostéoadhérine, des sialoprotéines (ostéopontine ou sialoprotéine osseuse BSP).

19. Utilisation selon la revendication 17, **caractérisée en ce que** lesdits protéoglycans sont choisis dans le groupe formé par:
a) l'aggrécane,
b) la versicane,
c) le biglycane,
d) la décorine.

20. Utilisation selon l'une des revendications 2 - 7 ou 9 - 19, **caractérisée en ce que** ledit agent est à administrer en combinaison avec des protéines sériques, de préférence choisies dans le groupe formé par:
a) l'albumine,
b) l'alpha-2HS glycoprotéine.

21. Utilisation selon l'une des revendications 2 - 7 ou 9 - 20, **caractérisée en ce que** ledit agent est à administrer en combinaison avec des enzymes, de préférence choisies dans le groupe formé par:
a) des métalloprotéinases,
b) des collagénases,
c) des gélatinases,
d) des stromélysines,
e) des activateurs du plasminogène,
f) des cystéine protéinases,
g) des protéinases aspartiques.

22. Utilisation selon l'une des revendications 2 - 7 ou 9 - 20, **caractérisée en ce que** ledit agent est à administrer en combinaison avec des sels de calcium, de la moelle osseuse ou de l'hydroxyapatite.

23. Utilisation selon l'une des revendications 2 - 7 ou 9 - 21, **caractérisée en ce que** ledit agent est à administrer en combinaison avec des sels de fluor, de préférence du fluorure de sodium ou du fluorophosphate monosodique.

24. Utilisation selon l'une des revendications 2 - 7 ou 9 - 23, **caractérisée en ce que** ledit agent est à administrer en combinaison avec des peptides, de préférence choisis dans le groupe formé par:
a) l'amyline,
b) des agents vasoactifs,
c) des neuropeptides.

25. Utilisation selon l'une des revendications 2 - 7 ou 9 - 24, **caractérisée en ce que** ledit agent est à administrer en combinaison avec des antioxydants, de préférence choisis dans le groupe formé par:
a) la cystéine,
b) la N-acétylcystéine,
c) des glutathions,
d) les vitamines A, C, D ou E.

26. Utilisation selon l'une des revendications 2 - 7 ou 9 - 25, **caractérisée en ce que** ledit agent est à administrer en combinaison avec une substance choisie parmi:
a) la transferrine,
b) le sélénium,
c) le bore,
d) le silicium,
e) des oxydes nitriques.

27. Utilisation selon l'une des revendications 1 ou 3 - 8 ou 11 - 26, **caractérisée en ce que** lesdites cellules osseuses sont choisies dans le groupe formé par les ostéoblastes, les cellules périostales, les cellules stromales de la moelle osseuse, les cellules satellites du tissu musculaire et des cellules souches mésenchymales.

28. Utilisation selon l'une des revendications 1 ou 3 - 8 ou 11 - 26, **caractérisée en ce que** lesdites cellules cartilagineuses sont choisies dans le groupe formé par les chondroblastes, les chondroclastes et les cellules souches mésenchymales.

29. Utilisation selon l'une des revendications 7 - 8 ou 11 - 28, **caractérisée en ce que** lesdites cellules osseuses ou cartilagineuses sont cultivés en monocouche, en culture en micromasse ou dans des structures tridimensionnelles biodégradables.

30. Utilisation selon la revendication 29, **caractérisée en ce que** ladite culture desdites cellules osseuses ou cartilagineuses dans ladite structure tridimensionnelle biodégradable génère un assemblage tridimensionnel de cellules possédant la structure d'une membrane, d'une mousse ou d'une éponge ensemencée.

31. Utilisation selon la revendication 29 ou 30, **caractérisée en ce que** la concentration dudit composé de créatine dans le milieu de culture est de l'ordre de 10 à 20 mM.

32. Utilisation selon l'une des revendications 29 à 31, **caractérisée en ce que** le milieu de culture contient de 0,1% à 5,0%, de préférence de 0,5% à 2% de sérum de veau foetal.

33. Utilisation selon l'une des revendications 29 à 32, **caractérisée en ce que** le milieu de culture contient de 10 à 250 µg, de préférence de 25 à 100 µg d'acide ascorbique ou d'une quantité équivalente d'un ascorbate pharmaceutiquement acceptable.

34. Utilisation selon l'une des revendications 29 à 33, **caractérisée en ce que** ladite culture de cellules est ensemencée avec 2.000 à 100.000 cellules, de préférence avec 10.000 à 50.000 cellules.

35. Utilisation selon l'une des revendications 1 à 34, **caractérisée en ce que** ledit composé de créatine ou ledit agent sont essentiellement exempts de dihydrotriazine.

36. Utilisation selon l'une des revendications 1 à 35, **caractérisée en ce que** ledit composé de créatine ou ledit agent sont essentiellement exempts de dicyanodiamide.

37. Utilisation selon l'une des revendications 1 à 36, **caractérisée en ce que** ledit composé de créatine ou ledit agent sont essentiellement exempts de créatinine en tant que produit de dégradation naturelle de la créatine.

38. Utilisation selon l'une des revendications 2 - 7, 9 - 26 ou 35 - 37, **caractérisée en ce que** ledit agent est à administrer aux patients humains en une quantité de 1,4 à 285 mg/kg par jour.

39. Utilisation selon l'une des revendications 2 - 6, 9 - 26 ou 35 - 37, **caractérisée en ce que** ledit agent est à administrer aux patients humains en une quantité de 0,1 à 20,0 g par jour.

40. Utilisation selon l'une des revendications 1 à 39, **caractérisée en ce que** ledit analogue de la créatine ou l'agent présente la formule générale:
Z₁ === C (=== Z₂) === X - A -Y
ainsi que ses sels pharmaceutiquement acceptables, où:
(a) Y est choisi dans le groupe formé par: -CO₂H, -NHOH, -NO₂, -SO₃H, -C(=O)NHSO₂J et -P(=O)(OH)(OJ), où J est choisi dans le groupe formé par: l'hydrogène, un radical alkyle à chaîne linéaire en C₁ à C₆, un radical alkyle ramifié en C₃ à C₆, un radical alcényle linéaire en C₂ à C₆, un radical alcényle ramifié en C₃ à C₆ et un radical aryle,
(b) A est choisi dans le groupe formé par: C, CH, des radicaux alkyle en C₁ à C₅, alcényle en C₂ à C₅, alkynyle en C₂ à C₅ et alcoyle en C₁ à C₅, chacun d'entre eux possédant de 0 à 2 substituants choisis indépendamment dans le groupe formé par:
(1) K, où K est choisi dans le groupe formé par: des radicaux alkyle linéaires en C₁ à C₆, alcényle linéaire en C₂ à C₆, alcoyle linéaire en C₁ à C₆, alkyle ramifié en C₃ à C₆, alcényle ramifié en C₃ à C₆, alcoyle ramifié en C₄ à C₆, K possédant de 0 à 2 substituants choisis indépendamment dans le groupe formé par des radicaux bromo, chloro, époxy et acétoxy,
(2) un radical aryle choisi dans le groupe formé par: un carbocycle à 1 - 2 noyaux et un hétérocycle à 1 - 2 noyaux, où le groupe aryle contient de 0 à 2 substituants choisis indépendamment dans le groupe formé par -CH₂L et -COCH₂L, où les L sont choisis indépendamment dans le groupe formé par des radicaux bromo, chloro, époxy et acétoxy, et
(3) -NH-M, où M est choisi dans le groupe formé par: l'hydrogène, des radicaux alkyle en C₁ à C₄, alcényle en C₂ à C₄, alcoyle en C₁ à C₄, alkyle ramifié en C₃ à C₄, alcényle ramifié en C₃ à C₄, et alcoyle ramifié en C₄ à C₆,
(c) X est choisi dans le groupe formé par: NR₁, CHR₁, CR₁, O et S, où R₁ est choisi dans le groupe formé par:
(1) l'hydrogène,
(2) K, où K est choisi dans le groupe formé par: des radicaux alkyle linéaires en C₁ à C₆, alcényle linéaires en C₂ à C₆, alcoyle linéaires en C₁ à C₆, alkyle ramifiés en C₃ à C₆, alcényle ramifiés en C₃ à C₆ et alcoyle ramifiés en C₄ à C₆, K possédant de 0 à 2 substituants choisis indépendamment dans le groupe formé par des radicaux bromo, chloro, époxy et acétoxy,
(3) un radical aryle choisi dans le groupe formé par: un carbocycle à 1 - 2 noyaux et un hétérocycle à 1 - 2 noyaux, où le groupe aryle contient de 0 à 2 substituants choisis indépendamment dans le groupe formé par -CH₂L et -COCH₂L, où les L sont choisis indépendamment dans le groupe formé par des radicaux bromo, chloro, époxy et acétoxy,
(4) un acide α-amino-ω-méthyl-ω-adénosylcarboxylique en C₅ à C₉ attaché via le carbone du ω-méthyle,
(5) un acide α-amino-ω-aza-ω-méthyl-ω-adénosylcarboxylique en C₅ à C₉ attaché via le carbone du ω-méthyle, et
(6) un acide α-amino-ω-thia-ω-méthyl-ω-adénosylcarboxylique en C₅ à C₉ où A et X sont liés par une liaison simple ou double,
(d) Z₁ et Z₂ sont choisis indépendamment dans le groupe formé par: =O, -NHR₂, -CH₂R₂, -NR₂OH, où Z₁ et Z₂ ne peuvent représenter tous deux =O et où R₂ est choisi dans le groupe formé par:
(1) l'hydrogène,
(2) K, où K est choisi dans le groupe formé par: des radicaux alkyle linéaires en C₁ à C₆, alcényle linéaires en C₂ à C₆, alcoyle linéaires en C₁ à C₆, alkyle ramifiés en C₃ à C₆, alcényle ramifiés en C₃ à C₆ et alcoyle ramifiés en C₄ à C₆, K possédant de 0 à 2 substituants choisis indépendamment dans le groupe formé par des radicaux bromo, chloro, époxy et acétoxy,
(3) un radical aryle choisi dans le groupe formé par: un carbocycle à 1 - 2 noyaux et un hétérocycle à 1 - 2 noyaux, où le groupe aryle contient de 0 à 2 substituants choisis indépendamment dans le groupe formé par -CH₂L et -COCH₂L, où les L sont choisis indépendamment dans le groupe formé par des radicaux bromo, chloro, époxy et acétoxy,
(4) un acide α-aminocarboxylique en C₄ à C₈ attaché via le carbone ω,
(5) B, où B est choisi dans le groupe formé par: -CO₂H, -NHOH, -NO₂, -SO₃H, -C(=O)NHSO₂J et -P(=O)(OH)(OJ), où J est choisi dans le groupe formé par: l'hydrogène, ou un radical alkyle linéaire en C₁ à C₆, alkyle ramifié en C₃ à C₆, alcényle linéaire en C₂ à C₆, alcényle ramifié en C₃ à C₆ et aryle, où B est éventuellement lié à l'azote via un groupe de liaison choisi dans le groupe formé par des radicaux alkyle en C₁ à C₂, alcényle en C₂ et alcoyle en C₁ à C₂,
(6) -D-E, où D est choisi dans le groupe formé par des radicaux alkyle linéaires en C₁ à C₃, alkyle ramifiés en C₃, alcényle linéaires en C₂ à C₃, alcényle ramifiés en C₃, alcoyle linéaires en C₁ à C₃, et aryle, et E est choisi dans le groupe formé par -(PO₃)ₙNMP, où n a une valeur de 0 à 2 et NMP est un ribonucléotide monophosphate lié via le 5'-phosphate, le 3'-phosphate ou le noyau aromatique de la base, -[P(=O)(OCH₃)(O)]ₘ-Q, où m a une valeur de 0 à 3 et Q est un ribonucléoside lié via le ribose ou le noyau aromatique de la base, -[P(=O)(OH)(CH₂)]ₘ-Q, où m a une valeur de 0 à 3 et Q est un ribonucléoside lié via le ribose du noyau aromatique de la base, et un radical aryle contenant de 0 à 3 substituants choisis indépendamment dans le groupe composé de: Cl, Br, époxy, acétoxy, -OG, -C(=O)G et -CO₂G, où G est indépendamment choisi dans le groupe formé par des radicaux alkyle linéaires en C₁ à C₆, alcényle linéaires en C₂ à C₆, alcoyle linéaires en C₁ à C₆, alkyle ramifiés en C₃ à C₆, alcényle ramifiés en C₁ à C₆, alcoyle ramifiés en C₄ à C₆, où E peut être attaché en n'importe quel point à D, et si D est un radical alkyle ou alcényle, D peut être lié à l'une ou aux deux extrémités par une liaison amide, et
(7) -E-, où E est choisi dans le groupe formé par -(PO₃)ₙNMP, où n a une valeur de 0 à 2 et NMP est un ribonucléotide monophosphate lié via le 5'-phosphate, le 3'-phosphate ou le noyau aromatique de la base, -P(P(=O)(OCH₃)(O))ₘ-Q, où m a une valeur de 0 à 3 et Q est un ribonucléoside lié via le ribose ou le noyau aromatique de la base, -[P(=O)(OH)(CH₂)]ₘ-Q, où m a une valeur de 0 à 3 et Q est un ribonucléoside lié via le ribose du noyau aromatique de la base et un groupe aryle contenant de 0 à 3 substituants choisis indépendamment dans le groupe formé par Cl, Br, époxy, acétoxy, -OG, -C(=O)G et -CO₂G, où G est indépendamment choisi dans le groupe formé par des radicaux alkyle linéaires en C₁ à C₆, alcényle linéaires en C₂ à C₆, alcoyle linéaires en C₁ à C₆, alkyle ramifiés en C₃ à C₆, alcényle ramifiés en C₃ à C₆, alcoyle ramifiés en C₄ à C₆, et si E est un radical aryle, E peut être lié par une liaison amide,
(e) si R₁ et au moins un groupe R₂ sont présents, R₁ peut être lié par une liaison simple ou double à un groupe R₂ pour former un cycle pentagonal à heptagonal,
(f) si deux groupes R₂ sont présents, ils peuvent être liés par une liaison simple ou double pour former un cycle pentagonal à heptagonal, et
(g) si R₁ est présent et que Z₁ ou Z₂ est choisi dans le groupe formé par -NHR₂, -CH₂R₂ et -NR₂OH, R₁ peut être lié par une liaison simple ou double au carbone ou à l'azote de Z₁ ou de Z₂ pour former un cycle tétragonal à heptagonal.

41. Utilisation selon l'une des revendications 2 - 7 ou 9 - 40, **caractérisée en ce que** ledit composé de créatine ou ledit agent sont utilisés pour le traitement de maladies dégénératives des cartilages, en particulier de l'arthrite.

42. Utilisation selon l'une des revendications 1 à 41, **caractérisée en ce que** lesdits composés de créatine ou ledit agent sont combinés à une transfection de cellules osseuses ou cartilagineuses par la créatine kinase.

43. Utilisation selon l'une des revendications 2 - 6, 9 - 26 ou 35 - 42, **caractérisée en ce que** ledit agent est additionnée de substances véhicules pharmaceutiquement appropriées afin d'améliorer la biodisponibilité.

44. Utilisation selon la revendication 43, **caractérisée en ce que** ladite substance véhicule est choisie dans le groupe formé par des hydrates de carbone, en particulier des maltodextrines et / ou du dextrose.

45. Utilisation de créatine en combinaison avec une ingénierie génétique de cellules cartilagineuses ou osseuses transfectées afin de surexprimer l'enzyme créatine kinase, pour la préparation d'un agent pour le traitement de cellules et de tissus osseux ou cartilagineux, **caractérisée en ce que**
a) des cellules de formation d'os ou de cartilage, prélevées sur un individu sain ou sur un patient, sont mises en culture cellulaire en présence de créatine et transfectées par de l'ADN complémentaire codant pour des isoformes de la créatine kinase et traitées destinées à surexprimer des isoenzymes de la créatine kinase,
b) les cellules métaboliquement modifiées obtenues à l'étape a) sont alors développées et cultivées pour former in vitro des tissus cartilagineux ou osseux génétiquement modifiés qui peuvent être transplantés dans des sites de lésions cartilagineuses ou osseuses dudit individu sain ou dudit patient.
